(19) Europäisches Patentamt
European Patent Office
Office européen des brevets

(11) Publication number: **0 505 605 A2**

(12) **EUROPEAN PATENT APPLICATION**

(21) Application number: **91105802.2**

(51) Int. Cl.5: **C12Q 1/68**, C12N 15/11

(22) Date of filing: **11.04.91**

The applicant has subsequently filed a sequence listing and declared, that it includes no new matter.

(30) Priority: **28.03.91 US 676292**

(43) Date of publication of application: **30.09.92 Bulletin 92/40**

(84) Designated Contracting States: **AT BE CH DE DK ES FR GB GR IT LI LU NL SE**

(71) Applicant: **WAYNE STATE UNIVERSITY**
**380 Mackenzie Hall**
**Detroit Michigan 48202(US)**

(72) Inventor: **Duncan, Craig H.**
**21508 Chestnut Lane**
**Farmington, Michigan 48024(US)**
Inventor: **Solus, Joseph F.**
**210 W. Maple**
**Ferndale, Michigan 48220(US)**
Inventor: **Kaplan, David J.**
**11729 15 Mile Road**
**Sterling Heights, Michigan 48077(US)**

(74) Representative: **Wharton, Peter Robert et al**
**Urquhart-Dykes & Lord Alliance House 29-31 Kirkgate**
**Bradford West Yorkshire, BD1 1OB(GB)**

(54) **Method and probes for detecting overlaps among large collections of cloned DNA molecules.**

(57) A method and probes for detecting overlaps among cloned DNA molecules includes the steps of hybridizing a labeled low frequency repetitive sequence probe with cloned DNA molecules, at least some of the cloned DNA containing overlaps including low frequency repetitive sequences, and identifying labeled hybridized molecules or fragments of molecules containing the low frequency repetitive sequences as including DNA from the same genomic location.

EP 0 505 605 A2

## TECHNICAL FIELD

The present invention relates to the physical mapping of DNA. More specifically, the present invention provides a method of detecting overlaps among cloned DNA molecules and further provides a probe of genetic material for detecting the overlaps.

## BACKGROUND OF THE INVENTION

A DNA map of a genome such as a physical map of the human genome may be an important research tool. A map of the human genome can be used to locate the relative position of genes, to assist in the study of hereditability of genetic diseases and can be used to validate a genetic map. The DNA map includes land marks which are genes or restriction enzyme recognition sites, the distance between sites being calculated in numbers of base pairs.

DNA maps can have either a genetic or a physical basis and offer various degrees of resolution.

The present invention relates to methods and tools for constructing a physical map.

There are 2.5 x $10^9$ base pairs of DNA in the haploid genome of each human cell. The DNA molecules exist in the form of 23 chromosomes which contain an average 1-2 x $10^8$ base pairs of DNA apiece. The DNAs of intact chromosomes are too complex to be analyzed at the molecular level. This requires that the DNA molecules be broken into smaller pieces which are then cloned in microorganisms, such as bacteria, bacteriophage or yeast. With present day technology, fragments of up to 4 x $10^4$ base pairs can be cloned in bacterial vectors known as cosmids (1). In yeast cells larger recombinant DNA molecules have been constructed containing approximately 10 times the cloning capacity of cosmid vectors (2). There is a gap of two to three orders of magnitude (100-1000 fold) between chromosome size DNA and the fragments of DNA which could be cloned by present day technology. At every point where chromosomal DNA is broken up to be packaged in clonable size fragments, there is a loss of information about the linear order and orientation of these fragments on the chromosome. The information must be reconstituted. This is often done by finding overlapping clones which between them cover all of the break points and the ends of the cloned DNA molecules. The search is a difficult process, which depends on locating favorable clones and on the random nature of the cloning procedures. Much of the labor is spent identifying overlapping segments shared by two or more clones. This is commonly done by obtaining limited structural information from the clones, either by determining short stretches of the DNA base sequence or by comparing restriction enzyme digestion patterns. Once two clones are overlapped, this forms a structure known as a contig (from contiguous clones). Once a contig is established, additional structural information from the sequence is used to detect overlaps with other clones or other contigs.

By its nature, this search is a pairwise procedure and a sequential procedure. The need for pairwise comparison means that the difficulty of finding overlaps increases as the square of the length of the DNA region which is being analyzed. The requirement for sequential overlapping means that many time-consuming steps must be done one after another, leading to unsuccessful delays in finishing large scale projects. Because of these reasons, methods for detecting overlaps which work for typical genetic loci (up to $10^5$ base pairs) rapidly become too cumbersome for detecting overlaps in DNA regions as large as chromosomes.

Novel families of interspersed repetitive elements from the human genome have been reported (7,24). Six novel families of interspersed repetitive elements have been detected in the available DNA sequences using computer assisted analysis. The estimated total number of elements in the reported six families is over 17,000. Sequences representative for each family range from approximately 150 to 650 base pairs in length and are predominately (A+T) rich (7). The inventors of the present invention have found eleven additional families of novel interspersed repetitive sequences in the human genome.

Seventeen of these novel families , referred hereafter as low frequency repetitive sequences, are utilized herein by the present invention to identify overlaps among cloned DNA molecules. Applicant has further constructed and utilized novel probes referred to as LF1-LF8, LF12, LF15-LF22 in accordance with the inventive method.

## SUMMARY OF THE INVENTION

In accordance with the present invention, there is provided a method of detecting overlaps among cloned DNA molecules, the method including the steps of hybridizing labeled low frequency repetitive sequence probes with cloned DNA molecules, at least some of the cloned DNA molecules containing overlaps including the low frequency repetitive sequences, and identifying molecules or fragments of molecules containing the low frequency repetitive sequences as including DNA from the same genomic location.

The present invention further provides a set of probes of genetic material for detecting the overlaps among the cloned DNA molecules, the probes including purified and labeled DNA fragments consisting of sequences of base pairs of DNA repeated about 300 to 10,000 times in the human genome. The sequences have substantially random distribution throughout the human genome. The probes are capable of detecting specific sites in the human genomic DNA.

## FIGURES IN THE DRAWINGS

Other advantages of the present invention will be readily appreciated as the same becomes better understood by reference to the following detailed description when considered in connection with the accompanying drawings wherein:

Figure 1 shows autoradiographs of nylon membranes containing equal plating densities of bacteriophage recombinant for a human genomic DNA library, the autoradiographs demonstrating the variance in copy number (per fraction of human genome) of several sequences recognized by different probes, each membrane being hybridized independently with the following probes, from top left and clockwise, human globin probe (as a low copy number control), LF1, LF3, and a Human Alu repeat probe, (as a high copy number control);

Figure 2 is a hybridization of mammalian chromosomal DNAs with novel repetitive DNA sequence, one ug samples of restriction endonuclease digested chromosomal DNAs being electrophoresed in 0.8% agarose gel arid blotted onto a nitrocellulose membrane, the membrane being incubated with a radiolabeled LF1 probe. Shown is an autoradiograph of the blot, the lanes being: M, molecular weight markers; 1, Human DNA digested with EcoR1; 2, Human DNA digested with BamH1; 3, Human DNA (different individual than #1 & 2) digested with EcoR1; 4, Cow DNA digested with EcoR1; 5, Mouse DNA digested with EcoR1; 6, Baboon DNA digested with EcoR1; 7, Macaque DNA digested with EcoR1;

Figure 3 demonstrates the analysis of clones from the centromere region of human chromosome 1 performed with the LF1 sequence probe, the probe hybridizing to six of the 190 clones arrayed on the grid, one of the clones hybridizing weakly to the probe. An ethidium bromide stained agarose electrophoresis gel of EcoR1 digests of those cloned DNA samples illustrates that the samples in lanes 2, 3, and 5 share portions of their restriction fragment pattern, those clones making up a contig of overlapping cloned DNA, the overlap among these 3 clones being large since much of the cloned DNA is present on fragments shared among the 3 samples, lanes 4 and 6 containing identical patterns from duplicated clones, there being no overlap among the restriction fragments in lane 1, lanes 2,3 and 5, and lanes 4 and 6 except for the fragment contributed by the cosmid vector itself, the LF1 probe having detected 3 sites where the LF1 repetitive sequence is present in the cloned minichromosome DNA, the last panel showing an autoradiograph of the transferred DNA from the gel to a membrane and then hybridized to the LF1 probe, a single band of identical mobility being visible in lanes 2, 3, and 5 (top arrow), it corresponding to one of the bands shared in lanes 2, 3, and 5 in the ethidium bromide stained gel. The lower arrow indicates the fragment which contains the LF1 sequence in the duplicated clone (lanes 4 and 6). The faint band (unmarked by arrow) in lane 1 indicates the band which contains the sequence in the clone which hybridized weakly to the LF1 probe in panel 1, Figure 1; and

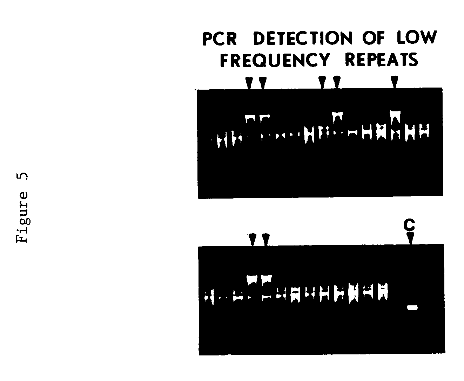

Figure 4 shows a contig which contains less overlapping DNA than the contig described in Figure 1, this contig having been identified by hybridization to the LF2 sequence probe. The figure shows the two clones which hybridize to LF2, a HindIII restriction digest of DNA from these clones, and the autoradiograph of the transferred DNA which illustrates the fragment that contains the LF2 sequence which hybridizes to the probe.

## DETAILED DESCRIPTION OF THE INVENTION

A method of detecting overlaps among cloned DNA molecules in accordance with the present invention generally includes the steps of hybridizing a labeled low frequency repetitive sequence probe with cloned DNA molecules, at least some of the cloned DNA molecules containing overlaps including the low frequency repetitive sequence, and then identifying molecules or fragments of molecules containing the low frequency repetitive sequences as including DNA from the same genomic location.

More specifically, the invention utilizes labeled low frequency repetitive sequence probes. The method critically depends upon the inventive suitable set of repetitive DNA sequence probes. Important characteristics of such probes are: 1) their repetition frequency, 2) their distribution in the human genome, 3) their number, and 4) their ability to detect specific sites in cloned genomic DNA.

With regard to repetition frequency of the low frequency probes constructed in accordance with the present invention, the repetitive DNA sequences are present in hundreds to thousands of copies per haploid genome. That is, the probes include purified and labeled DNA fragments consisting of sequences of base pairs of DNA repeated about 300 to 10,000 times in the human genome. The fragments may be labeled by various means, such as by radiolabel or fluorescent label.

The repetition frequency of these sequences is a key factor with regard to the subject invention. Several classes of highly repetitive interspersed DNA sequences have been described in the human genome, the most numerous being in the Alu family (3). Another class is the L1 elements (4). Both of these families are too numerous to yield information of the present invention because almost every clone of length greater than a few kilobases would be expected to contain one of these two families of repeats.

In contrast, the low frequency probes described herein have a proper repetition frequency to effectively divide a complex set of clones into easily handled subsets. It is this capacity of the DNA fragments from a genome to be divided easily into subsets which is critical to the present invention.

The distributions of the low frequency sequences in the human genome are critical. The low frequency sequence probes cannot be used to map regions of the genome in which they are not present. Accordingly, the overall utility of the present invention in the construction of physical maps in all areas of the genome and the ability to use the present invention to close maps in these areas depends upon whether there is exclusion or under representation of the sequences in regions of the human genome. The representation of low frequency probes described both in GenBank sequence data base (representing approximately 0.15% of the human genome) and in a fragment of the human genome used in the present application as a test for the utility of this mapping method (representing approximately 0.125% of the human genome) is consistent with the repetition frequencies determined for the low frequency probes described herein. Available information indicates that there is a random distribution of these sequences throughout similar areas of the human genome.

The evidence for this is found in examining the chromosomal locations for members of low frequency repeat families which are present in the vicinities of known mapped genes. The known chromosomal locations of LF sequences are as follows:

| LF | GenBank designation | gene locus | chr |
|---|---|---|---|
| 1 | HUMFIXG 8000-8268 | blood clotting factor IX | X |
| 1 | HUMB2M2 1863-2030 | $\beta$2 microglobulin | 15 |
| 1 | HUMHMGA14A 8044-8248 | high mobility group protein HMG-14 | 21 |
| 2 | HUMTPA 22931-23031 | tissue plasminogen activator | 8 |
| 2 | HUMTFPB 8913-8989 | tissue factor $\beta$ | ? |
| 3 | HUMCRYGBC 9067-9180 | gamma crystallin | 2 |
| 4 | HUMFOLA2 12-64 | dihydrofolate reductase | 5 |
| 4 | HUMTPA 34152-35153 | tissue plasminogen activator | 8 |
| 5 | HUMTPA 5307-6297 | tissue plasminogen activator | 8 |
| 5 | HUMTPA 24498-25144 | tissue plasminogen activator | 8 |
| 5 | HUMCRYGBC 4720-4877 | gamma crystallin | 2 |
| 6 | HUMAGG 727-981 | angiogenin | 14 |
| 6 | HUMAPOAI1 6055-6395 | apolipoprotein AI | 11 |
| 6 | HUMTPA 40-578 | tissue plasminogen activator | 8 |
| 6 | HUMPAIA 1-511 | plasminogen activator inhibitor | ? |
| 7 | HUMFIXG 6669-7025 | blood clotting factor IX | X |
| 7 | HUMERYA 2165-2478 | erythrocyte alpha spectrin | 1 |
| 7 | HUMTPA 18392-18851 | tissue plasminogen activator | X |
| 7 | HUMAPOA4C 1-145 | apolipoprotein A4C | 11 |
| 8 | HUMFIXG 24021-24170 | blood clotting factor IX | X |
| 8 | HUMNGFB 3399-3550 | nerve growth factor $\beta$ | 1 |
| 12 | HUMCYP345 2880-3063 | cytochrome P-3-450 | 19 |
| 12 | HUMHPRTB 37059-37280 | hypoxanthine phosphoribosyl xferase | X |
| 12 | HUMBCR221 1090-1220 | BCR gene | 22 |
| 12 | HUMTPA 17474-17877 | tissue plasminogen activator | 8 |
| 16 | HUMATP1A2 4820-4980 | Na-K ATPase subunit | 2 |
| 16 | HUMLMWOS1 390-540 | oligoadenylate synthetase | ? |
| 17 | HUMSIGMG3 2000-2150 | IGc(gamma)3 | 14 |
| 17 | HUMINT2 11440-11560 | int2 proto-oncogene protein | 12 |
| 17 | HUMMBP1A 273-400 | myelin basic protein | 18 |
| 17 | HUMC21DLA 1600-1800 | long arm DNA chrom 21 | 21 |
| 17 | HUMHPRTB 54772-54975 | hypoxanthine phosphoribosyl xferase | X |
| 18 | HUMSEXREPB 350-560 | sex chromosome repeat | X |
| 18 | HUMPADP 45-240 | amyloid A4 protein | 21 |
| 18 | HUMC21DLA 1395-1560 | long arm DNA chrom 21 | 21 |
| 18 | HUMHPRTB 54960-55145 | hypoxanthine phosphoribosyl xferase | X |
| 18 | HUMHPRTB 7812-8071 | hypoxanthine phosphoribosyl xferase | X |
| 19 | HUMRASSK2 530-750 | SK2 c-HA-ras-1 oncogene protein | 11 |
| 20 | HUMRPS17A 3245-3500 | ribosomal protein S17 | 5 |
| 21 | HUMCYAR01 1010-1120 | aromatase cytochrome P-450 | 15 |
| 22 | HUMHXAMUT 1-150 | hexose aminidase | 15 |
| 22 | HUMIL2RBA 170-320 | interleukin-2 receptor $\beta$ chain | 10 |

**Legend.** Chromosomal locations for members of low frequency repeat (**LF**) families. Each occurrence of a low frequency repeat family is listed. The **GenBank designation** refers to GenBank locus names and sequence numbering. **Gene locus** refers to the common name of the gene or gene product. **Chr** refers to the chromosomal location of the locus (if known).

This data implies that low frequency repeats, even members of the same family, are randomly distributed among the various human chromosomes in accordance with the requirements of this invention.

Applicant herein specifically sets forth seventeen different low frequency sequence probes constructed by applicant. The more probes which are available, the more overlaps that can be detected in a bank of clones. More importantly, the more probes there are available, the easier it is to link the smaller contigs (contiguous clones) together. Applicant shows herein that the isolation of a large number of low frequency repetitive DNA probes can be accomplished. Applicant's laboratory has developed several of these probes in a screen of just 0.1% of the human genome.

The ability of the low frequency repetitive sequence probes to detect specific sites in genomic DNA is critical. The ability of a particular sequence to recognize other similar sequences in the genome is tested below by commonly used hybridization methods. This does not exclude the possibility of changing the hybridization conditions to recognize a larger or smaller number of sites in the genome by any given probe. It is also possible for the inventive probes disclosed herein to recognize particular sites in the genome by methods other than conventional hybridization techniques. These sequences are considered as flags capable of marking particular sequence sites in the genome. The method is successful when at least some of the cloned DNA contains overlaps including the low frequency repetitive sequences. In accordance with the inventive method, a labeled low frequency repeat is hybridized with a collection , or library, of cloned DNA molecules. A library is considered to be a population of microorganisms containing overlapping cloned DNA molecules which cover a certain region of the genome under study. The invention is applicable to finding overlaps in any library, ranging from libraries covering the entire genome to libraries covering only a small fraction of a chromosome. However, it is envisioned that in practice the library will typically represent either a single chromosome or a fragment of a chromosome.

As the first step, the microorganisms composing the library are transferred and fixed to a membrane support. This membrane is then incubated with labeled low frequency probes in situ to determine which microorganisms contain representatives of a low frequency repeat family. An independent hybridization experiment is performed for each low frequency probe. Thus, each probe will select a subset of the library composed of those cloned DNA molecules which contain a member of the repeat family to which that probe belongs.

The second step of the invention is to identify overlapping clones within the selected subsets. In general the goal is to organize the selected subset into groups which overlap at a genomic site containing a specific member of the low frequency repeat family. The number of groups which is generated is equal to the number of different members of the repeat family represented in the original library. This identifying step can be accomplished by purifying DNA molecules from the selected subsets of the library and subjecting these purified DNA molecules to further tests.

For example, the purified DNA molecules can be digested with a restriction enzyme. The resulting fragments are separated by size by gel electrophoresis and than transferred to a hybridization membrane (Southern blot analysis, 15). The membrane is then incubated with the labeled low frequency probe followed by autoradiography. This procedure will determine the sizes of the DNA restriction fragments which contain the genomic locations of each member of the low frequency repeat family. Overlapping clones will possess their low frequency repeat sequences on identical size restriction fragments, with some exceptions. The information from these experiments can thus be used to organize the clones into overlapping groups. The exceptions referred to above occur when a given chromosomal restriction fragment was truncated during the original packaging of the chromosome into clonable size fragments. This limitation can be overcome by digesting the purified DNA, in independent experiments, with different restriction enzymes. Unless the chromosomal location of the low frequency repeat is very close to the truncation point of the clone, many restriction enzymes will be found to generate identical size restriction fragments between overlapping clones in the Southern blot analysis.

A further advantage of this method, which is of particular benefit when Yeast Artificial Chromosomes (YACs) are used as the cloning vector, is that it does not require that the cloned DNA molecules be separated from the endogenous genomic DNA of the microorganisms which are used as the host. This is because the Southern blot analysis is capable of yielding size information about the hybridizing fragments even in the presence of a large excess of extraneous DNA molecules. The only condition required is the absence of sequences in the endogenous genomic DNA of the microorganisms which hybridize to the low frequency repeat probes. That these conditions are met is shown in Figure 2, which idicates that the low frequency probes used here are limited in their natural distribution to primate DNAs.

Thusly, two criteria are examined. One, whether or not a given clone contains a member of a low frequency repeat family and secondly, whether the restriction fragments are of identical size. Identical size restriction fragments indicate that the two clones include the same genomic location marked by the low frequency and therefore represent overlapping regions. Thusly, clones containing DNA from the particular genomic location will identify a contig (from contiguous clones) whose length is limited to twice the cloning capacity of the vector used to construct the recombinant clones. In this manner, overlaps among cloned DNA molecules are determined.

Alternative methods can be used, such as specifically sequencing the subset of initially hybridized clones containing probes as opposed to the enzymatic digestion step. However, when working with large initial subsets of clones, the second hybridization step greatly reduces the number of possibilities to be examined and provides a high probability indicator correlating the common size fragments including the low frequency repetitive sequences as containing overlapping genomic locations.

Large numbers of clones can be screened in parallel with the labeled probes made from different low frequency repetitive DNA families. The DNAs from each set of clones containing a particular repetitive sequence are analyzed to determine which clones contain DNA from individual genomic locations. Once contigs have been identified, the next stage of the procedure is to find overlaps between the contigs. This method results in ordering of a plurality of fragments, the ordering producing a physical map of the genomic material.

Generally, once several clones are screened in parallel with several labeled low frequency repetitive sequence probes, molecules or fragments containing two or more low frequency sequences are identified. The fragments sharing common genomic locations identified by molecules or fragments sharing different low frequency repetitive sequences with different molecules or fragments are ordered. This is most easily done by examining those subsets of clones which hybridize to two or more of the low frequency repetitive sequence probes. These subsets will have already been identified by comparing the data between the initial screens with individual repetitive DNA sequence probes as described above. Each member of a subset will have been mapped into two contigs during the previous procedure because of identified genomic locations of two different repetitive sequence elements. In other words, those two contigs have been linked into a single contig by the clone which hybridized both sequence probes. The size of the contig identified by a linking clone will depend upon the distance between the two different repetitive sequences on the linking clone. The greater the size of the clone, the greater the information provided by the linking step. The number of contigs linked together will depend upon the density of these repetitive sequence elements in the human genome and the cloning capacity of the vector used. It is expected that the density of low frequency repeats will be adequate for this task. Two known genetic loci, the tissue plasminogen activator gene and the blood clotting factor IX gene, contain seven and three low frequency repeats respectively.

Instructive comparisons can be reached by considering the results of following the described protocol when using other types of probes. High frequency repeating probes such as the Alu would require fewer experiments but would provide little information because almost every clone contains an Alu repeat. Single copy (non-repetitive) probes would require an independent experiment for genomic region corresponding to the average size of a cloned fragment. This would require 50,000-100,000 experiments to map the whole genome cloned in cosmid vectors. In contrast, the same resolution could be achieved with 50-100 different low frequency repetitive sequence probes of average 1000 copies each. Such probes constructed in accordance with the present invention provide for a minimum number of experiments while maintaining sufficient variability to identify different overlapping genomic regions.

The parallel procedure described above is readily amenable to automation or computer processing. The hybridization analysis performed with each repetitive sequence is equivalent to the same procedure performed sequentially with a large number of nonrepetitive sequence probes.

As an illustration of the present invention, low frequency repetitive sequence probes constructed in accordance with the present invention have been tested for their application in genome mapping by using cloned sequences isolated from a chromosome fragment which originated from the centromere region of the human chromosome 1 (5,6). An array of 321 recombinant cosmid clones grown on the surface of nylon membranes was screened by hybridization to the low frequency repetitive sequenced probes described herein. These clones represent 11,000 kilobase (kb) of human DNA sequence from a portion of the genome 8,000 kb in length. This prototype study represents a rigorous test of the proposed mapping method for three reasons: 1) a total of only 90% of the length of the human chromosome fragment is represented in the number of recombinants screened. Therefore the potential for overlap among these cloned sequences is not large because of the limited sample size. 2) The average size of DNA cloned in the cosmid vector described herein is 35 kb. Yeast artificial chromosome vectors are readily capable of carrying ten times that amount of DNA. The larger the cloning capacity of the vector used, the easier it is to identify and link contigs by the method described herein. 3) The region of the human genome under study represents the centromere region. This area has been considered as a problematic region for mapping because of the presence of satellite DNA. The presence of low frequency repetitive sequences in this region which can be used to recognize contiguous clones is an argument for the overall utility of the mapping method of the present invention.

METHODS

Construction of Probes:

Seventeen probes have been used for this invention. All of these probes were constructed in applicant's laboratory. The sequence information used to construct these probes came from two sources. Twelve probes, LF1, LF2, LF3, LF12, LF15, LF16, LF17, LF18, LF19, LF20, LF21, LF22 were constructed using information from a screening method which was developed in applicant's laboratory and described below. Five probes, LF4-8, were constructed using information derived from a computer analysis of DNA databases as described by Jurka (7).

Screening Method (For LF1, LF2,LF3, LF19, LF20, LF21):

Human genomic DNA was digested to completion with the restriction enzyme AluI. The resulting fragments were fractionated on a 7% polyacrylamide gel and fragments in the 500-1000 bp region were isolated. 50 ng of this size fractionated DNA was ligated to 500 ng of SmaI cleaved M13mp19 RF DNA (8). The vector was phosphatased before use. The ligated DNA was mixed with competent JM109 bacteria (purchased from Stratagene Inc., San Diego, CA) and 10,000 resulting transformants were plated at a density of 1,650 plaques per 10 cm petri dish. Duplicate filter replicates were prepared and were incubated separately with either of two radioactive oligonucleotide probes. One of these was the 25 mer GTGGCTCA-[C/T][A/G]CCTGTAATCCCAGCA; the other was the

33 mer GG[C/T]TGCAGTGAGC[C/T][A/G][T/A]GAT

[C/T][A/G][C/T][A/G]CCA[C/T]TCGACT.

Both of these were derived from the Alu family consensus sequence (9). Oligonucleotides were synthesized by Research Genetics Inc. (Huntsville, AL). Phage which hybridized to both probes were plated at lower density and subjected to a repeat screening with the same probes. Single stranded template DNA was extracted from small scale (2ml) cultures of these phage.

Screening Method (For LF12, LF15, LF16, LF17, LF18, LF22).

One μg of genomic human placental DNA was mixed with 20 μg each of two oligonucleotides. One oligonucleotide was the 31 mer
GGGTCGACAGTGAGCCGAGATCGCGCCACTG; the second oligonucleotide was the 28 mer
GGGGATCCTGGATTACAGGCGTGAGCC. The reaction mixture was adjusted to a volume of 200μl, containing each dNTP at a final concentration of 300μM, polymerase reaction buffer, and 40 units of Thermus aquaticus DNA polymerase (Amersham). The polymerase chain reaction was performed for 25 cycles of 94° for three minutes, followed by 50° for five minutes followed by 72° for five minutes. The reaction owas then extracted twice with an equal volume of phenol, once with an equal volume of CHC13, and ethanol precipitated. The DNA was then dissolved to 50 μl of restriction enzyme buffer, followed by the addition of 25 units of SalI and 50 units of BamH1. After a four hour incubation at 37°, the reaction mixture was heated at 65° for five minutes and DNA fragments were purified by two cycles of preparative electrophoresis on a 7% polyacrylamide gel. DNA fragments in the 300-1000 bp region were isolated. 40 ng of this fractionated DNA was ligated to 100 ng of BamH1, Sal1 cleaved M13mp19 RF DNA (from which the 12bp Sal1-Bam-H1 insert had been removed by S-300 gel filtration [Pharmacia]). The ligated DNA was mixed with competent JM109 bacteria (Stratagene Inc.) and 2000 resulting transformants were plated at a density of 150 plaques per 10 cm petri dish on NZY plates containing β-galactosidase indicator dye. Duplicate filter replicates were prepared and were incubated separately with either of two radioactive probes. One probe was a 69 bp sequence taken from the internal region of an Alu repeat

(CACTTTGGGAGGCCAAGGCAGGTGGATCACCTCAAGTCAGGAGTTCAAGGC

CAGCCTGACCAACATGGA).

The second probe was a 5 kb fragment containing an L1 sequence from the region 5' to the human γ-gamma globin gene. 431 phage plaques were selected by the criteria of not reacting with the dye indicator and not hybridizing to either of the radioactive probes. Single stranded template DNA was extracted from small scale (2ml) cultures of these phage.

DNA sequencing and analysis (For LF1, LF2, LF3, LF12, LF15, LF16, LF17, LF18, LF19, LF20, LF21, LF22).

The single stranded templates were sequenced by the dideoxy termination method (10) as modified (11) using T7 DNA Polymerase (12). 600-700 bp of sequence information was obtained from each template. The sequence data were analyzed using the IBI-Pustell software purchased from International Biotechnologies Inc. to find homologous sequences in the GenBank DNA Database (Release 59). Sequences which showed homology to database sequences were used to make probes as described in the next section.

DNA subcloning, amplification, and hybridization (For LF1-LF8, LF12, LF15-LF22):

The DNA sequence information was used to construct oligonucleotides which flank the repetitive DNA elements in known examples of cloned human DNA molecules. These oligonucleotides were incubated with the human DNA molecules under conditions appropriate for amplification as described (13) using a Perkins-Elmer-Cetus DNA thermocycler. Resulting DNA fragments were subcloned in the Bluescript vector (Stratagene Inc) and transformed into the E.coli strgin XLI-blue (Stratagene Inc.). After purification of the plasmid molecules, the probe DNAs were liberated by digestion with EcoR1 (for probes LF2-LF8, LF12, LF15-LF22) or HindIII and BamH1 (for probe LF1). Then the DNA fragments containing the probe sequences were purified by preparative gel electrophoresis on 7% polyacrylamide non-denaturing gels. DNA Southern blot analysis of genomic DNA was performed as described (14,15) under conditions of reduced stringency. Hybridizations were performed in 6xSSC 20mM NaP04 (pH7.0), 50% Formamide, 10% Dextran sulfate, 0.5% SDS at 39`C for 6-18 hours. Then the membranes were washed twice for 5 minutes in 1xSSC, 0.5% SDS at room temperature, followed by two washes of 15 minutes each in 0.2 x SSC, 0.5% SDS at 45`C. In situ plaque hybridizations (16) and random primer labeling of DNA (17) were performed as described.

Cloning the human minichromosome in recombinant cosmids:

A genomic library has been reconstructed using the human-Chinese hamster hybrid X12.1.3(5,6) and the cosmid vector pWE-15(18) in applicant's laboratory. Cosmid DNA was digested to completion with BamH1, treated with bacterial alkaline phosphatase, subjected to extraction against phenol-chloroform, and collected by precipitation in the presence of ethanol. Cosmid DNA was rehydrated at approximately 1ug/ul TE buffer, its concentration determined by comparison of a diluted sample to known amounts of lambda DNA in an ethidium bromide stained agarose gel, and its concentration adjusted to 500ng/ul.

High molecular weight DNA prepared from the hybrid X12.1.3 was digested with MboI under conditions observed to yield the largest number of fragments in 35-45 kb size range. The DNA was extracted against phenol-chloroform, collected by precipitation, and rehydrated at an estimated concentration of 750 to 1500 ug/ml TE. Approximately 300ul of sample was fractionated on a 10-40% sucrose step-gradient by centrifugation for 18 hours at 25,000rpm in a Beckman SW-27 rotor. Fractions of approximately 500ul were collected from the bottom of the centrifuge tube, dialyzed against 0.1 x TE, (10mM Tris pH 8.0 1mM EDTA) concentrated by isobutanol extraction, extracted against phenol-chloroform, and precipitated in the presence of ethanol. Precipitates from individual fractions were rehydrated in 15ul TE, and 1ul samples were compared to known amounts of lambda DNA in an ethidium bromide stained agarose gel.

As individual fractions, 10ng of MboI digested X12.1.3 DNA were ligated to 500ng of BamH1 digested, phosphatase treated pWE-15 DNA in a 10ul volume in the presence of 0.3u T4 DNA ligase at 15°C overnight. The DNA in 1ul of each ligation reaction was packaged into lambda phage particles which were used to infect DK-1 cells. The X12.1.3 DNA fraction which produced the largest number of colonies was used to scale up the ligation, packaging and infection procedures. A library of 5.2 x $10^5$ recombinant cosmids was amplified by overnight growth on agar dishes with yeast tryptone (YT) growth medium containing 30mg/ml kanamycin and 10 mM MgS04 at a density of 8 x $10^3$ colonies/150cm diameter plate. Cells were harvested from the surfaces of the plates, resuspended in YT medium containing 15% glycerol, and stored frozen at -70°C.

The amplified library has been screened for minichromosome DNA by hybridization to human DNA sequences. Between 5 x $10^3$ and 2 x $10^4$ colonies per 150mm diameter plate were transferred to Whatman 541 paper and examined for the presence of human DNA sequences by hybridization at 37°C in hybridization solution containing 30% formamide. Total human genomic DNA and cloned alphoid satellite sequences (19,20) were used to detect potential human DNA-containing recombinants.

The bacteria in the areas of the plates showing hybridization to human DNA were streaked to form single colonies. Colonies were picked, grown as patched cultures, transferred to Whatman 541, and again hybridized to human sequences in hybridization solution containing 30% formamide, but at 45° C. Recombinants showing hybridization to human DNA under these conditions were picked and grown to prepare frozen cell stocks and DNA stocks by the alkaline lysis technique (21).

Approximately 1-2 ug of each recombinant cosmid DNA sample was digested with EcoR1, PstI or HindIII in order to examine the quality of the DNA preparations. Digestions were usually done in microtiter plates. A visual comparison of restriction endonuclease patterns among the isolated recombinants allowed the elimination of duplicate clones.

Preparation of grids of cloned minichromosome DNA-containing recombinant cosmids:

Cloned minichromosome DNA samples were organized into a grid-like array for subsequent hybridization experiments. Dilute bacterial cell stocks of the minichromosome DNA containing clones (stored frozen in 15% glycerol/YT medium) were distributed to individual wells of sterile microtiter plates for subsequent storage and use. An inoculum of the samples in the wells of the microtiter plates was aseptically transferred to the surface of nylon hybridization membranes (Amersham Hybond N) with a device containing 96 vertical stainless steel pins anchored to a steel plate. The samples from a number of microtiter plates can be transferred to a single membrane by carefully offsetting the positions of the subsequent inocula. The inoculated membranes were placed upon the surface YT agar containing 50 ug/ml ampicillin and incubated at 37° C overnight.

Membranes were peeled from the surface of the agar and floated in shallow trays on the surface of 1.5M NaCl, 0.5M NaOH for 10 minutes, then 1M TrisHCl pH 8.0 for 5 minutes, and finally 3M NaCl, 0.5M TrisHCl, pH 7.0 for 10 minutes. Membranes were allowed to dry slightly and each was treated with 5ml chloroform. Membranes were exposed to a short wave UV source for 2 minutes (Fotodyne DNA Transfer Lamp). Dry filters were stored at room temperature between sheets of filter paper for future use.

Identification of clones containing low frequency repetitive sequences:

Hybridization membranes containing 190-321 cloned minichromosome DNA samples arranged in a grid pattern were incubated for 3-18 hours at 60° C in prehybridization buffer (500mM NaCl, 50mM NaPhosphate pH 7.0, 5mM EDTA, 5x Denhardt's solution, 1% SDS, 100ug/ml sonicated herring sperm DNA), then for 15-20 hours in hybridization buffer (prehybridization buffer containing 10% dextran sulfate and 25-100ng 32P labeled denatured sequence probe). Membranes were incubated in 2x SSC, 0.1% SDS at 45° C, 50° C, and 55° C until much of the radioactivity had been washed from the surface of the filters. Membranes were rinsed in 2x SSC (0.3M Nacl 0.03M Na Citrate pH 8.0) at room temperature and mounted moist between plastic wrap in film cassettes with intensifier screens. Kodak XOMAT AR-5 film was exposed to the membranes.

Southern transfers (14,15), nick translation labeling reactions (22), and random oligo primed labeling reactions (23) were performed as described.

The polymerase chain reaction was used to screen samples of 29 different cosmid DNAs for the presence of LF1 repeats. Two outward facing primers were used. The primers were taken from the DNA sequence

TTTATACAGTCATGCATCTCTTAA-TGGGGATGCGTTCTGA-

AAATGAGCCCTTAGGCAGTTTTATTG-TGTGAGC-C-

TCATAGAGTGCACTTATGCAAAC-TAG.

The polymerase chain reactin was performed (27) using the Thermus flavus DNA polymerase (from NEN-DuPont), according to the manufacturer's specifications. Reaction products were electrophoresed on a 1% agarose gel, stained with ethidium, and photographed under UV light. Clones testing positive are marked by arrowheads. The lane marked 'c' is a control template.

RESULTS

Origins of the low repetitive sequence probes:

The probes used in these examples are referred to by the numbers LF1-LF8, LF12, LF15-LF22. A brief description of the origins and sequence of these probes follows:

LF1 (XL1-blue/pCDLF1 deposited at the ATCC, having designator 68254) - This probe is taken from Kaplan and Duncan et al. (24) (the sequence marked ANO in Fig. 1).

LF2 (XL1-blue/pCDLF2 deposited at the ATCC, having designator 68255) - This probe is taken from unpublished data from applicant's laboratory. Its sequence is

```
        10         20         30         40         50         60
TCCATGTACT CAACATATAC TCAAGTCCTG CAGTCAGCCC CACAGAACCT GCATATATGG
        70         80         90        100        110        120
CTAGTTTGCA TCCTGCAAAT ATTGTATTTT CAATCCACAT TTGATTGAAA AAGTCTGCAT
       130        140        150        160        170        180
GTAAGTCGAC CTGTGCAGTT CAAACCCATG TTGTTCAAGG GTCAACTATG TATGAAAACA
       190        200        210        220        230        240
TTTCTCTTCA GTAAGTACTT AGAAGTGGAA
```

LF3 (XL1-blue/pCDLF3 deposited at the ATCC, having designator 68257 - This probe is taken from unpublished data from applicant's laboratory. Its sequence is

```
        10         20         30         40         50         60
TCCTAGGCCT TCGCATTTAC TCACCACTCA CTGACTCAGT CAGAGCAACT TCCAGTCCTG
        70         80         90        100
CAAGCTCCAT TTATGGTAAG TACCCTATAC AGGTGTACCA TT
```

LF4 (XL1-blue/pCDLF4 deposited at the ATCC, having designator 68257) - This probe is taken from the sixth intron of the human Tissue Plasminogen Activator gene (bases #31172 to 31592 in Fig. A, ref. 25).

LF5 (XL1-blue/pCDLF5 deposited at the ATCC, having designator 68258) - This probe is taken from the sixth intron of the human Tissue Plasminogen Activator gene (bases #21195 to 21481 in Fig. A, ref 25).

LF6 (XL1-blue/pCDLF6 deposited at the ATCC, having designator 68259) - This probe is taken from the region 5' to the human Tissue Plasminogen Activator gene (bases #-3491 to -2965 in Fig. A, ref 25).

LF7 (XL1-blue/pCDLF7 deposited at the ATCC, having designator 68260) - This probe is taken from the first intron of the human gene for blood clotting factor IX (bases #3719 to 4050 in Fig. 3, ref 26).

LF8 (XL1-blue/pCDLF8 deposited at the ATCC, having designator 68261) - This probe is taken from the sixth intron of the human gene for blood clotting factor IX (bases #21815 to 21991 in Fig. 3, ref 26).

LF12 This probe is taken from an unidentified locus in the human genome according to unpulished data from applicant's laboratory. Its sequence is,

```
        10         20         30         40         50         60
CAGTGGCACC CAGCCCCATT TTAACCATTT TTAAGTGGAC AGTTCACTGA CATTAAGTAC
        70         80         90        100        110        120
TACACATTCG CATTGTTGTG CCACCATCCC CACCATCCAT CTCCAGAACT TTTTTATCTT
       130        140        150        160        170        180
GTGAAACTAC GACTCTATAC TCCTTTAACA ATAACTCTCC ATTCTACCCT CTCCACAGCC
       190        200        210        220        230        240
CCTAGCAACT ACCATTCTAC TTTCTGTGTC TATAAATTTG ACTATTCTAA GTACCTCATA
       250
TGA
```

LF15 This probe is taken from an unidentified locus in the human genome according to unpublished data from applicant's laboratory. Its sequence is,

```
        10         20         30         40         50         60
CAGCTGTACT GGGCTGATAA GGGCCAATTC TAATGGGCTC ATCTTAACTT GATGACATCC
        70         80         90        100        110        120
ACAAAGTCTC TGTTTCCAAA TAAGGTCACA TTCACAGGCA CCAGAAGGTA GGGCTTCAAT
       130        140        150        160        170
ATGCATTTTG GGCGACAGAC TTCACCCACT GACAGCCATA TGTGTTCTTT ACAGTTG
```

LF16 This probe is taken from an unidentified locus in the human genome according to unpublished data from applicant's laboratory. Its sequence is,

```
        10         20         30         40         50         60
CAGCTGCATC TGGCCTAAAT CCTAATGTAA ACTATGAATT TTGGGTGATA ATGATGTGTA
        70         80         90        100        110        120
AATATAGGTT CATCAATTGT AACAAAGGTA CTACTTTGGT TCAGATGTTG ATAGTGGATA
       130        140        150        160
AGGCTCTGCA TGTATGTCTA GGAAAGGAGT ATATGGGTTA TCTCTG
```

LF17 This probe is taken from an unidentified locus in the human genome according to unpublished data from applicant's laboratory. Its sequence is,

```
        10         20         30         40         50         60
GGCCCACCCT ACTCCAGTAC GACCTCATCT GGGTTAATTC CTCGTGTGAT GGCCCTGTTT
        70         80         90        100        110        120
CCAAATGGTC ACAAACTGAG GTTCTAGGGG GTTAGGTCTT GAACGTATGA ATTTTGGGGA
       130        140        150        160
AATATGATGT ACCCCATAAC ACAAGGCATT TGAGACCTTA TTG
```

LF18 This probe is taken from an unidentified locus in the human genome according to unpublished data from applicant's laboratory. Its sequence is,

```
        10         20         30         40         50         60
CAGCTGTGCC CAGCCTGTTT TTAGTCTTAT AGTGGCATTC TGGGGCTCTT GTGTTGTAGT
        70         80         90        100        110        120
GATATTGAGT TGCACATTGT CTTAGTTTCC TAGGCTACCT TAACGAAGTA CCACAGACTG
       130        140        150        160        170        180
AGTTACTTAA ACAACAGAAA CTTATTTTTG TACAATTCTG GAGACTGGAA GTCAAGATCA
       190        200        210        220        230        240
AGGTGCTGGA GGGATTAGTT TCTCTCGAGG TCTCCTTGGC TTGTAGATGC TGTCTTTTTC
       250        260        270        280
CTGTGTCTAC ACAATGCCTT CCTCTGTGCA TGTCTGTGA
```

LF19 This probe is taken from an unidentified locus in the human genome according to unpublished data from applicant's laboratory. Its sequence is,

```
        10         20         30         40         50         60
TGATTAGATG GTGTCCACTC AGATTAAGGG TGGGTCTGCC TTTCCCCAGC CCACTGAGTC
        70         80         90        100        110        120
CAATGTTAAC CTCCTTTGGC AACACCCTCA CAGACACACC CAGGATCAAT ACTTTGTAAC
       130        140        150        160        170        180
CTTCAATCTA ATAAAGTTGA CAGTATTAAC CATCACACCT GCCTAAGTAA AGATTAAATG
       190        200
AGATGATCAT CACAACGCAT ATTTG
```

LF20 This probe is taken from an unidentified locus in the human genome according to unpublished data from applicant's laboratory. Its sequence is,

```
        10         20         30         40         50         60
CTTGTAAGCA GGGCTTTCAA AAGGCAGCAT TTGCATCTGC TATGTTAACT TTTTAATACG
        70         80         90        100        110        120
CTTTGGGCAG AGAACTGTAA GAGATCCCAG AGGACATTTG ACAATTTCTG GAGACATTTC
       130        140        150        160        170        180
TGTTGTCGTA ACTGGGAGGT CCACAGACAT ACATTGGATA GAAGCCAGGA ATGCTGCTAA
       190        200        210        220        230        240
ATGCTATGCA GTCCACAGAA CAGCCCCCAT CCCCTCAACC AAAAATTATG TGGCCCAAAA
       250        260        270        280        290        300
TGTCAGTAGT GTCAAGGTGG AGAAACTCTC GTCTAGACCT ACCCTAACAG AATTTAACAA
       310        320
CAAGCCTGGA AGGATCAAGT TGA
```

LF21 This probe is taken from an unidentiifed locus in the human genome according to unpublished data from applicant's laboratory. Its sequence is,

```
        10         20         30         40         50         60
AATTCTGACA CTGTCTACCT GGAGATAGCA TCAAATCCCA GGGTTGAGGT CTCAGTCTGA
        70         80         90        100        110        120
CAAGACTGCA CCCCTCTTCA GACACAGTAG CATGTCTGGG CCTCTGGAAC TTCTGACCAA
       130        140        150        160        170        180
CTGGCTTCAC ATTGGGGTAA CCAGACTTCG TATTTATGTT CAATTAATTT GCTAGAGTGG
       190        200        210        220        230        240
CTCACAAAAC TCAGAGAAAT ACATTTACTG GTTAATTATA AAGGATATTT ATGCCTTTAT
       250        260        270        280        290        300
ATTCCTTGCA CTTTCAATTC ATCTGTTTCT TTCAGGCACAT CTTTATAGA A
```

LF22 This probe is taken from an unidentified locus in the human genome according to unpublished data from applicant's laboratory. Its sequence is,

```
        10         20         30         40         50         60
CAGCCGCGCC CGGCCTATAT ATATATTTCT GGATGACGTG AGATATTTTG ATACAGGCAT
        70         80         90        100        110        120
GCAATCGATA ATAATCACAT CAAGGTAAAT GAGGTCTCCA TCCCCGCAAT CATTTATCCT
       130        140        150        160        170        180
TTCTGTTACA AATGATCCAG TCTACTCTTG TAG
```

Repetition frequency of the probes (For LF1-LF8, LF12, LF15-LF22):

Subcloned DNA fragments were prepared and radioactively labeled as described. These labeled DNA fragments were used as probes for in situ hybridization with 21,000 human recombinant bacteriophage plaques (16). Each bacteriophage contained an average of $1.5x10^4$ bases of cloned human DNA. Accordingly, this collection of phage contained $1.5x10^4 x2.1x10^4 = 3.15x10^8$ bases of DNA. The haploid human genome contains $2.5x10^9$ bases of DNA. $2.5x10^9/3.15x10^8 = 7.94$. Therefore, for each probe, the number of hybridizing bacteriophage plaques (determined by autoradiography) was multiplied by 7.94 to determine the repetition frequencies per haploid genome.

Each probe was tested for repetition frequency and the results are presented in Table 1. Some representative autoradiographs for two of the LF probes are shown in Figure 1. The sequences of the LF probes are repeated 320-6700 times in the human haploid genome based upon hybridization analysis. These repetition frequencies are consistent with the number of times the sequences are found in the GenBank database which represents approximately 0.15% of the human genome.

Southern blotting to show repetitive nature:

LF1 was used as a probe in a genomic Southern blot of genomic human and other mammalian DNAs. The results shown in Figure 2 show that this probe hybridizes to primate DNAs but not DNA from other mammals.

Identification of contiguous clones from the centromere region of chromosome 1:

These repetitive probes are being tested for their applications to genome mapping by using cloned sequences isolated from a human chromosome fragment which originated from the centromere region of human chromosome 1. The existence of this region as a minichromosome in a human-Chinese hamster hybrid cell line has been described (5,6). Human DNA from this minichromosome has been isolated as recombinant cosmid clones as described in the Method section herein.

Labeled LF probes were hybridized to grids of minichromosome DNA-containing recombinants to determine which clones contained individual LF sequences. All LF sequence probes described above to clones present on the grid (Table 2: #clones detected in mini). 199 of the 321 clones represented on the grid contain at least one of the seventeen LF sequences tested. Thus, the LF sequences are distributed in the human genome in a way that includes the presence of these sequences on a portion of the human genome representing approximately 0.125% of the total.

In order to determine whether or not the LF sequence probes had detected any contigs, the DNA cloned within the recombinants which hybridized to a particular LF probe were compared by examination of their restriction endonuclease digest patterns. DNA from those clones which hybridized to a particular probe were cleaved with a restriction endonuclease, the resulting fragments were separated by agarose gel electrophoresis, and the fragment patterns were visualized by ethidium bromide staining and UV illumination. The patterns of restriction fragments from the cloned insert DNAs were compared to determine if portions of the restriction fragment patterns were shared among the clones which hybridized to a particular LF sequence probe.

For samples which appeared to contain overlaps, the DNA in the gel was transferred to hybridization membrane. Hybridization was performed on the Southern transfers as it had been done for the colony grid transfers to see if the sequence responsible for the cross hybridization was present upon a fragment shared by the different clones. Examples of these analyses are shown in Figures 3 and 4 and the data summarized in Table 2.

Approximately 7000 kb of the cloned minichromosome DNA represented on the grid (199 of 321 recombinant cosmids) hybridizes to a LF repetitive sequence described. Furthermore 29% of the LF recombinant cosmids hybridize to two different LF sequences, making them good candidates to link small contigs into larger ones. Thus, by using only seventeen LF sequence probes in this limited test system, the distribution of these sequences in this part of the human genome, the identification of contigs by hybridization, and the existence of LF sequences in proximity to act as links between contigs has been demonstrated.

The polymerase chain reaction was used to detect the presence of the low frequency repeats LF1 in cloned DNA, thereby representing an alternative method of hybridization. The sequence of this region, in double stranded format, is shown below. The primers used in this experiment are marked by double underlines:

```
        10         20         30         40         50         60
         *          *          *          *          *          *
TGCATCTCTTAATGGGGATGCGTTCTGAAAATGAGCCCTTAGGCAGTTTTATTGTGTGAG
ACGTAGAGAATTACCCCTACGCAAGACTTTTACTCGGGAATCCGTCAAAATAACACACTC

        70         80         90        100        110        120
         *          *          *          *          *          *
CCTCATAGAGTGCACTTATGCAAACTAGATGGTATAGCCTACACACACCTAGGCTTTGTG
GGAGTATCTCACGTGAATACGTTTGATCTACCATATCGGATGTGTGTGGATCCGAAACAC

       130        140        150        160        170        180
         *          *          *          *          *          *
ATAGAGCCTACTGCTCGTAGGCTACAAGCTGTACAGCATGTTACTGTACTGAATACCATA
TATCTCGGATGACGAGCATCCGATGTTCGACATGTCGTACAATGACATGACTTATGGTAT

       190        200        210        220        230        240
         *          *          *          *          *          *
GGCAGTAACAATGGTAAGTATTTGTGTATCTATACATAGAAATGGTTAAGTAAAAATATA
CCGTCATTGTTACCATTCATAAACACATAGATATGTATCTTTACCAATTCATTTTTATAT

GAATAGG
CTTATCC
```

Accordingly, applicant presents herein a novel method and tools for use with the method for constructing a physical map of a genome. More specifically, the present invention provides tools for first detecting overlaps among cloned DNA molecules and further utility of the tools for ordering a plurality of DNA fragments including overlapping base pair portions.

The present inventive method depends vitally upon a suitable set of repetitive DNA sequence probes constructed in accordance with the present invention. These repetitive sequences are short in length (less than 1000 base pair in length) and are present in hundreds to thousands of copies per haploid genome.

The probes and examples discussed herein relate to mapping the human genome. The same probes could also be used to map the genomes of non-human higher primates, such as the chimpanzee, gorilla, baboon, or macaque.

In addition, this invention includes in its scope the development of probes and physical maps for any plant or animal genome which contains low frequency repeating sequences. The applicants contemplate isolating probes from and generating maps of animals which are important in research (mice) or commerce (cows, pigs, chickens, fish, sheep or goats) and also probes and maps of the genomes of plants.

SEQUENCE LISTING

(1) GENERAL INFORMATION:

(i) APPLICANT: Duncan, Craig H.
Solus, Joseph F.
Kaplan, David J.

(ii) TITLE OF INVENTION: METHOD AND PROBES FOR DETECTING OVERLAPS AMONG LARGE COLLECTIONS OF CLONED DNA MOLECULES

(iii) NUMBER OF SEQUENCES: 21

(iv) CORRESPONDENCE ADDRESS:
(A) ADDRESSEE: Reising, Ethington, Barnard, Perry & Milton
(B) STREET: P.O. Box 4390
(C) CITY: Troy
(D) STATE: Michigan
(E) COUNTRY: U.S.A.
(F) ZIP: 48099

(v) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25

(vi) CURRENT APPLICATION DATA:
(A) APPLICATION NUMBER: EP 91105802.2
(B) FILING DATE: 11-APR-1991
(C) CLASSIFICATION:

(viii) ATTORNEY/AGENT INFORMATION:
(A) NAME: Kohn, Kenneth I.
(B) REGISTRATION NUMBER: 30,955
(C) REFERENCE/DOCKET NUMBER: FP-319(WSU) EPC

(ix) TELECOMMUNICATION INFORMATION:
(A) TELEPHONE: (313) 689-3554

(2) INFORMATION FOR SEQ ID NO:1:

(i) SEQUENCE CHARACTERISTICS:
(A) LENGTH: 247 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:
(F) TISSUE TYPE: Placenta

(vii) IMMEDIATE SOURCE:
(A) LIBRARY: M13
(B) CLONE: V11

(ix) FEATURE:
(A) NAME/KEY: repeat_region
(B) LOCATION: 1..247

(xi) SEQUENCE DESCRIPTION: SEQ ID NO:1:

TGCATCTCTT AATGGGGATG CGTTCTGAAA ATGAGCCCTT AGGCAGTTTT ATTGTGTGAG 60

CCTCATAGAG TGCACTTATG CAAACTAGAT GGTATAGCCT ACACACACCT AGGCTTTGTG 120

ATAGAGCCTA CTGCTCGTAG GCTACAAGCT GTACAGCATG TTACTGTACT GAATACCATA 180

GGCAGTAACA ATGGTAAGTA TTTGTGTATC TATACATAGA AATGGTTAAG TAAAAATATA 240

GAATAGG 247

(2) INFORMATION FOR SEQ ID NO:2:

(i) SEQUENCE CHARACTERISTICS:
(A) LENGTH: 280 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:
(A) ORGANISM: Homo sapiens
(D) DEVELOPMENTAL STAGE: Fetus
(F) TISSUE TYPE: Liver

(vii) IMMEDIATE SOURCE:
(A) LIBRARY: lambda charon 4A
(B) CLONE: lambda hM-17-2

(viii) POSITION IN GENOME:
(A) CHROMOSOME/SEGMENT: 10

(ix) FEATURE:
(A) NAME/KEY: repeat_region
(B) LOCATION: 1..280

(x) PUBLICATION INFORMATION:
(A) AUTHORS: PICADO-LEONARD, J.
MILLER, W. L.
(B) TITLE: CLONING AND SEQUENCE OF THE HUMAN GENE FOR P450C21 (STEROID 17 A-HYDROXYLASE/17,20 LYASE): SIMILARITY WITH THE GENE FOR P450C21.
(C) JOURNAL: DNA
(D) VOLUME: 6
(E) ISSUE: 5
(F) PAGES: 439-448
(G) DATE: 18-JUN-1987

(xi) SEQUENCE DESCRIPTION: SEQ ID NO:2:

TGTATGCGGG AAGTCAGGGA CCTGAATGAA GGGACTGCTG GAGCCATGGC AGAGGAACAT 60

AAATTGTGAA GATTTCATTT AATATGGACA TTTATCAGTT CCCAAATAAT ACTTTTATAA 120

TTTCTTATGC CTGTCTTTAC TTTAATCTCT TAATCCTGTT ATCTTTGTAA GCTGAGGATG 180

TTTGTCACTT CAGGACCACT GTGATAATTG TGTTAACTGT ACAAATTGAT TGTAAGACAT 240

GTGTTTGAAC AATATGAAAT TAGTGCACCT TGAAAAAGAA 280

(2) INFORMATION FOR SEQ ID NO:3:

(i) SEQUENCE CHARACTERISTICS:
(A) LENGTH: 186 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:
(A) ORGANISM: Macaca fascicularis
(D) DEVELOPMENTAL STAGE: Fetus
(F) TISSUE TYPE: Liver

(vii) IMMEDIATE SOURCE:
(A) LIBRARY: lambda charon 4
(B) CLONE: BAB-D

(ix) FEATURE:
(A) NAME/KEY: repeat_region
(B) LOCATION: 1..186

(x) PUBLICATION INFORMATION:
(A) AUTHORS: SAVATIER, P.
TRABUCHET, G.
CHEBLOUNE, Y
FAURE, C.
VERDIER, G.
NIGON, V. M.
(B) TITLE: NUCLEOTIDE SEQUENCE OF THE DELTA-BETA-GLOBIN INTERGENIC SEGMENT IN THE MACAQUE;STRUCTURE AND EVLOLUTIONARY RATES IN HIGHER PRIMATES.
(C) JOURNAL: J. Mol. Evol.
(D) VOLUME: 24
(E) ISSUE: 1
(F) PAGES: 297-308
(G) DATE: 1987

(xi) SEQUENCE DESCRIPTION: SEQ ID NO:3:

CACATGGACA CAAAGAAGAG ATCAACAGAC ATGCAGGTCT ACTTGAGGGT TGAGGGTGGG 60

AAGAGGGAGA GGATGAAAAA AGTACCTATT GGGTACTAAG TTTATTAGCT GAGTGATGAA 120

ATAATCTGTA CATCAAAACC CAGTGACATG CAATTTACCT ATATAAGTTG TACATGTACC 180

CCCAAA 186

(2) INFORMATION FOR SEQ ID NO:4:

(i) SEQUENCE CHARACTERISTICS:
(A) LENGTH: 166 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:
(A) ORGANISM: Homo sapiens
(F) TISSUE TYPE: Placenta

(vii) IMMEDIATE SOURCE:
(A) LIBRARY: M13
(B) CLONE: LGM10A

(ix) FEATURE:
(A) NAME/KEY: repeat_region
(B) LOCATION: 1..166

(x) PUBLICATION INFORMATION:
(A) AUTHORS: KAPLAN, DAVID J.
JURKA, JERZY
DUNCAN, CRAIG H.
(B) TITLE: MEDIUM REITERATION FREQUENCY REPETITIVE SEQUENCES IN THE HUMAN GENOME
(C) JOURNAL: Nucleic Acids Res.
(G) DATE: 24-APR-1991

(xi) SEQUENCE DESCRIPTION: SEQ ID NO:4:

CAGCTGCATC TGGCCTAAAT CCTAATGTAA ACTATGAATT TTGGGTGATA ATGATGTGTA 60

AATATAGGTT CATCAATTGT AACAAAGGTA CTACTTTGGT TCAGATGTTG ATAGTGGATA 120

AGGCTCTGCA TGTATGTCTA GGAAAGGAGT ATATGGGTTA TCTCTG 166

(2) INFORMATION FOR SEQ ID NO:5:

(i) SEQUENCE CHARACTERISTICS:
(A) LENGTH: 279 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:
(A) ORGANISM: Homo sapiens
(F) TISSUE TYPE: Placenta

(vii) IMMEDIATE SOURCE:
(A) LIBRARY: M13
(B) CLONE: IV-8

(ix) FEATURE:
(A) NAME/KEY: repeat_region
(B) LOCATION: 1..279

(x) PUBLICATION INFORMATION:
(A) AUTHORS: KAPLAN, DAVID J.
JURKA, JERZY
DUNCAN, CRAIG H.
(B) TITLE: MEDIUM REITERATION FREQUENCY REPETITIVE SEQUENCES IN THE HUMAN GENOME
(C) JOURNAL: Nucleic Acids Res.
(G) DATE: 24-APR-1991

(xi) SEQUENCE DESCRIPTION: SEQ ID NO:5:

```
CAGCTGTGCC CAGCCTGTTT TTAGTCTTAT AGTGGCATTC TGGGGCTCTT GTGTTGTAGT     60
GATATTGAGT TGCACATTGT CTTAGTTTCC TAGGCTACCT TAACGAAGTA CCACAGACTG    120
AGTTACTTAA ACAACAGAAA CTTATTTTTG TACAATTCTG GAGACTGGAA GTCAAGATCA    180
AGGTGCTGGA GGGATTAGTT TCTCTCGAGG TCTCCTTGGC TTGTAGATGC TGTCTTTTTC    240
CTGTGTCTAC ACAATGCCTT CCTCTGTGCA TGTCTGTGA                           279
```

(2) INFORMATION FOR SEQ ID NO:6:

(i) SEQUENCE CHARACTERISTICS:
(A) LENGTH: 210 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:
(A) ORGANISM: Homo sapiens
(F) TISSUE TYPE: Placenta

(vii) IMMEDIATE SOURCE:
(A) LIBRARY: M13
(B) CLONE: V63 ( v i i i)

(viii) POSITION IN GENOME:
(A) CHROMOSOME/SEGMENT: unknown
(B) MAP POSITION: unknown
(C) UNITS: unknown

(ix) FEATURE:
(A) NAME/KEY: repeat_region
(B) LOCATION: 1..210

(xi) SEQUENCE DESCRIPTION: SEQ ID NO:6:

```
TCCATGTACT CAACATATAC TCAAGTCCTG CAGTCAGCCC CACAGAACCT GCATATATGG     60
CTAGTTTGCA TCCTGCAAAT ATTGTATTTT CAATCCACAT TTGATTGAAA AAGTCTGCAT    120
GTAAGTCGAC CTGTGCAGTT CAAACCCATG TTGTTCAAGG GTCAACTATG TATGAAAACA    180
TTTCTCTTCA GTAAGTACTT AGAAGTGGAA                                     210
```

(2) INFORMATION FOR SEQ ID NO:7:

(i) SEQUENCE CHARACTERISTICS:
(A) LENGTH: 291 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:
(A) ORGANISM: Homo sapiens
(F) TISSUE TYPE: Placenta

(vii) IMMEDIATE SOURCE:
(A) LIBRARY: M13
(B) CLONE: IV-113

(ix) FEATURE:
(A) NAME/KEY: repeat_region
(B) LOCATION: 1..291

(xi) SEQUENCE DESCRIPTION: SEQ ID NO:7:

```
AATTCTGACA CTGTCTACCT GGAGATAGCA TCAAATCCCA GGGTTGAGGT CTCAGTCTGA     60
CAAGACTGCA CCCCTCTTCA GACACAGTAG CATGTCTGGG CCTCTGGAAC TTCTGACCAA    120
CTGGCTTCAC ATTGGGGTAA CCAGACTTCG TATTTATGTT CAATTAATTT GCTAGAGTGG    180
CTCACAAAAC TCAGAGAAAT ACATTTACTG GTTAATTATA AAGGATATTT ATGCCTTTAT    240
ATTCCTTGCA CTTTCAATTC ATCTGTTTCT TTCAGGCACA TCTTTATAGA A             291
```

(2) INFORMATION FOR SEQ ID NO:8:

(i) SEQUENCE CHARACTERISTICS:
(A) LENGTH: 102 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:
(A) ORGANISM: Homo sapiens
(F) TISSUE TYPE: Placenta

(vii) IMMEDIATE SOURCE:
(A) LIBRARY: M13
(B) CLONE: 3a-1

(ix) FEATURE:
(A) NAME/KEY: repeat_region
(B) LOCATION: 1..102

(xi) SEQUENCE DESCRIPTION: SEQ ID NO:8:

```
TCCTAGGCCT TCGCATTTAC TCACCACTCA CTGACTCAGT CAGAGCAACT TCCAGTCCTG     60
CAAGCTCCAT TTATGGTAAG TACCCTATAC AGGTGTACCA TT                       102
```

(2) INFORMATION FOR SEQ ID NO:9:

(i) SEQUENCE CHARACTERISTICS:
(A) LENGTH: 286 base pairs
(B) TYPE: nucleic acid

(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:
(A) ORGANISM: Homo sapiens
(D) DEVELOPMENTAL STAGE: Fetus
(F) TISSUE TYPE: Liver

(vii) IMMEDIATE SOURCE:
(A) LIBRARY: lambda charon 4A
(B) CLONE: pM14Pst2 3

(viii) POSITION IN GENOME:
(A) CHROMOSOME/SEGMENT: 8
(B) MAP POSITION: 8p12

(ix) FEATURE:
(A) NAME/KEY: repeat_region
(B) LOCATION: 1..286

(xi) SEQUENCE DESCRIPTION: SEQ ID NO:9:

```
TTCCAGGCCA TAGGTAGATA AGAGACAAAA GGCTGTATTC TGAGTCCTTG ATCAGCTTTT    60

CACTGAACAC ACAATTGAGT CTGGCTCAGT TCATCTGCAT TTTTACATAA AAAATAGGGC   120

AGAGGAAGCA ATCAGATACG CATTTGTCTC AGATGAGCAG AGGGATGATT TTCTGTCCTG   180

CACCTGGGAA GATAAGCTAT CCATTTACAA TGCCAAGGTG AAAGTCAACA GAACTGTTTT   240

AGGGTAAAGA TCTTTAGGCC TGCAAGGAAT GTCCTTGTAG AGAATT                  286
```

(2) INFORMATION FOR SEQ ID NO:10:

(i) SEQUENCE CHARACTERISTICS:
(A) LENGTH: 332 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:
(A) ORGANISM: Homo sapiens
(D) DEVELOPMENTAL STAGE: Fetus
(F) TISSUE TYPE: liver

(vii) IMMEDIATE SOURCE:
(A) LIBRARY: lambda charon 4A
(B) CLONE: FIXlambda4243

(viii) POSITION IN GENOME:
(A) CHROMOSOME/SEGMENT: X
(B) MAP POSITION: long arm

(ix) FEATURE:
(A) NAME/KEY: repeat_region
(B) LOCATION: 1..332

(xi) SEQUENCE DESCRIPTION: SEQ ID NO:10:

TCAGCTCTCC TTGTGAGTTC CACAGCCACA GATTCAATTA ACTGCAGATC AAAAATATTC 60

AAGAAAAAAA TGGATGGTTC GATCTCTACT GAACATGTAC AGACTCTTTT ATCTTTCATT 120

ATTCCCTAAA CAATACAGCA TAACAACTAT TTACATAGCA TTTACATTGT ATTAGCTATT 180

AAGAGAAACC TAGAGATGAT TTAAAGTACA AAGGAGGATG TGTTTAGGTT ATATGCAAAT 240

AGTAAGCCAT TTTTATATCG GAGACTTGAG CATCCACAGA TCTTGATATT TGCAGGGGGT 300

CTTGCCACCA ATTTTCCATG GATACTGAGG AA 332

(2) INFORMATION FOR SEQ ID NO:11:

(i) SEQUENCE CHARACTERISTICS:
(A) LENGTH: 378 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:
(A) ORGANISM: Homo sapiens
(D) DEVELOPMENTAL STAGE: Fetus
(F) TISSUE TYPE: liver

(vii) IMMEDIATE SOURCE:
(A) LIBRARY: lambda charon 4a
(B) CLONE: FIXlambda61

(viii) POSITION IN GENOME:
(A) CHROMOSOME/SEGMENT: x
(B) MAP POSITION: long arm

(ix) FEATURE:
(A) NAME/KEY: repeat_region
(B) LOCATION: 1..378

(xi) SEQUENCE DESCRIPTION: SEQ ID NO:11:

TTCAGAGCTG AGAGCCCCGA ACAGAGATTT ACCCACATAT TTATTGACAG CAAGCCAGTC 60

ATAAGATTTA CTGAAAGTAT TCCTTATGGG AAATAAAGGG ATGAGTCTGG CTAGTTATCT 120

GCAGCAGGAA CATGTCCTTA AGGCACAAAT CACTTATGCA ATTGTCTGTG GTTTAAGAAC 180

ACCTTTAAGC AGTTTTCCGC CCTGGGTGGG CCAGGTGTTC CTTGCCCTCA TTCTGGTAAA 240

CCCACAACCT TCCAGTGTGG ATATCAAGGC CATCACGAGC ATATCACAGT GCTGCAGAGA 300

TTTTGTTTAT GGCCAGTTTT GGGGCCAGTT TATGGCCAGA TTTGGAGGCC TGTTCCCAAC 360

AAACCAGAAG CTAGGAAT 378

(2) INFORMATION FOR SEQ ID NO:12:

(i) SEQUENCE CHARACTERISTICS:
(A) LENGTH: 281 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:
(A) ORGANISM: Homo sapiens
(E) HAPLOTYPE: HLA-A2, B27, Cw1, DR1, SB4
(G) CELL TYPE: lymphoblastoid
(H) CELL LINE: 3.1.0

(vii) IMMEDIATE SOURCE:
(A) LIBRARY: cosmid pJB8
(B) CLONE: SB-2

(viii) POSITION IN GENOME:
(A) CHROMOSOME/SEGMENT: 6
(B) MAP POSITION: 6p21.3

(ix) FEATURE:
(A) NAME/KEY: repeat_region
(B) LOCATION: 1..281

(xi) SEQUENCE DESCRIPTION: SEQ ID NO:12:

TCTGCAGGCT GTACGCGAAA CATGGCACTA GCATCTGCTT CTGTTGGGGG ATTCTGGAAG 60

CTTTTACTCA TGGTGGAAGG CAAGTGGAGC CAGTGCATCA CATGGTCATA GAGGGAGAAA 120

GAGACATAGA AAGAGGTGCC AGCCTCTTTT TAACAACCAG GTTTCATGTG CACTAATAGA 180

GTGAGAACTC ACTCATTACC CGGAGAGGGG ACAAAGCCAT TCATGAGGGT CTCCTCCATG 240

ATTCAAATAC CTCCCACCAG GCCCCACCTG CAACACTGGG G 281

(2) INFORMATION FOR SEQ ID NO:13:

(i) SEQUENCE CHARACTERISTICS:
(A) LENGTH: 243 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:
(A) ORGANISM: Homo sapiens
(D) DEVELOPMENTAL STAGE: Fetus
(F) TISSUE TYPE: Placenta

(vii) IMMEDIATE SOURCE:
(A) LIBRARY: M13mp19
(B) CLONE: LGN142

(ix) FEATURE:
(A) NAME/KEY: repeat_region
(B) LOCATION: 1..243

(xi) SEQUENCE DESCRIPTION: SEQ ID NO:13:

CAGTGGCACC CAGCCCCATT TTAACCATTT TTAAGTGGAC AGTTCACTGA CATTAAGTAC 60

TACACATTCG CATTGTTGTG CCACCATCCC CACCATCCAT CTCCAGAACT TTTTTATCTT 120

GTGAAACTAC GACTCTATAC TCCTTTAACA ATAACTCTCC ATTCTACCCT CTCCACAGCC 180

CCTAGCAACT ACCATTCTAC TTTCTGTGTC TATAAATTTG ACTATTCTAA GTACCTCATA 240

TGA 243

(2) INFORMATION FOR SEQ ID NO:14:

(i) SEQUENCE CHARACTERISTICS:
(A) LENGTH: 177 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:
(A) ORGANISM: Homo sapiens
(F) TISSUE TYPE: Placenta

(vii) IMMEDIATE SOURCE:
(A) LIBRARY: M13
(B) CLONE: LGN40A

(ix) FEATURE:
(A) NAME/KEY: repeat_region
(B) LOCATION: 1..177

(xi) SEQUENCE DESCRIPTION: SEQ ID NO:14:

CAGCTGTACT GGGCTGATAA GGGCCAATTC TAATGGGCTC ATCTTAACTT GATGACATCC 60

ACAAAGTCTC TGTTTCCAAA TAAGGTCACA TTCACAGGCA CCAGAAGGTA GGGCTTCAAT 120

ATGCATTTTG GGCGACAGAC TTCACCCACT GACAGCCATA TGTGTTCTTT ACAGTTG 177

(2) INFORMATION FOR SEQ ID NO:15:

(i) SEQUENCE CHARACTERISTICS:
(A) LENGTH: 163 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:
(A) ORGANISM: Homo sapiens
(F) TISSUE TYPE: Placenta

(vii) IMMEDIATE SOURCE:
(A) LIBRARY: M13
(B) CLONE: LGN158

(ix) FEATURE:
(A) NAME/KEY: repeat_region
(B) LOCATION: 1..163

(xi) SEQUENCE DESCRIPTION: SEQ ID NO:15:

GGCCCACCCT ACTCCAGTAC GACCTCATCT GGGTTAATTC CTCGTGTGAT GGCCCTGTTT 60

CCAAATGGTC ACAAACTGAG GTTCTAGGGG GTTAGGTCTT GAACGTATGA ATTTTGGGGA 120

AATATGATGT ACCCCATAAC ACAAGGCATT TGAGACCTTA TTG 163

(2) INFORMATION FOR SEQ ID NO:16:

(i) SEQUENCE CHARACTERISTICS:
(A) LENGTH: 205 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:
(A) ORGANISM: Homo sapiens
(F) TISSUE TYPE: Placenta

(vii) IMMEDIATE SOURCE:
(A) LIBRARY: M13
(B) CLONE: III-2

(ix) FEATURE:
(A) NAME/KEY: repeat_region
(B) LOCATION: 1..205

(xi) SEQUENCE DESCRIPTION: SEQ ID NO:16:

TGATTAGATG GTGTCCACTC AGATTAAGGG TGGGTCTGCC TTTCCCCAGC CCACTGAGTC 60

CAATGTTAAC CTCCTTTGGC AACACCCTCA CAGACACACC CAGGATCAAT ACTTTGTAAC 120

CTTCAATCTA ATAAAGTTGA CAGTATTAAC CATCACACCT GCCTAAGTAA AGATTAAATG 180

AGATGATCAT CACAACGCAT ATTTG 205

(2) INFORMATION FOR SEQ ID NO:17:

(i) SEQUENCE CHARACTERISTICS:

(A) LENGTH: 323 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:
(A) ORGANISM: Homo sapiens
(F) TISSUE TYPE: Placenta

(vii) IMMEDIATE SOURCE:
(A) LIBRARY: M13
(B) CLONE: III-168

(ix) FEATURE:
(A) NAME/KEY: repeat_region
(B) LOCATION: 1..323

(xi) SEQUENCE DESCRIPTION: SEQ ID NO:17:

```
CTTGTAAGCA GGGCTTTCAA AAGGCAGCAT TTGCATCTGC TATGTTAACT TTTTAATACG    60
CTTTGGGCAG AGAACTGTAA GAGATCCCAG AGGACATTTG ACAATTTCTG GAGACATTTC   120
TGTTGTCGTA ACTGGGAGGT CCACAGACAT ACATTGGATA GAAGCCAGGA ATGCTGCTAA   180
ATGCTATGCA GTCCACAGAA CAGCCCCCAT CCCCTCAACC AAAAATTATG TGGCCCAAAA   240
TGTCAGTAGT GTCAAGGTGG AGAAACTCTC GTCTAGACCT ACCCTAACAG AATTTAACAA   300
CAAGCCTGGA AGGATCAAGT TGA                                           323
```

(2) INFORMATION FOR SEQ ID NO:18:

(i) SEQUENCE CHARACTERISTICS:
(A) LENGTH: 153 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:
(A) ORGANISM: Homo sapiens
(F) TISSUE TYPE: Placenta

(vii) IMMEDIATE SOURCE:
(A) LIBRARY: M13
(B) CLONE: C8

(ix) FEATURE:
(A) NAME/KEY: repeat_region
(B) LOCATION: 1..153

(xi) SEQUENCE DESCRIPTION: SEQ ID NO:18:

CAGCCGCGCC CGGCCTATAT ATATATTTCT GGATGACGTG AGATATTTTG ATACAGGCAT 60

GCAATCGATA ATAATCACAT CAAGGTAAAT GAGGTCTCCA TCCCCGCAAT CATTTATCCT 120

TTCTGTTACA AATGATCCAG TCTACTCTTG TAG 153

(2) INFORMATION FOR SEQ ID NO:19:

(i) SEQUENCE CHARACTERISTICS:
(A) LENGTH: 419 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:
(A) ORGANISM: Homo sapiens
(D) DEVELOPMENTAL STAGE: Fetus
(F) TISSUE TYPE: Liver

(vii) IMMEDIATE SOURCE:
(A) LIBRARY: lambda charon 4A1
(B) CLONE: pM21Eco4

(viii) POSITION IN GENOME:
(A) CHROMOSOME/SEGMENT: 8
(B) MAP POSITION: 8p12

(ix) FEATURE:
(A) NAME/KEY: repeat_region
(B) LOCATION: 1..419

(xi) SEQUENCE DESCRIPTION: SEQ ID NO:19:

GGATGATGGC GGCCATAATA GTGGTGGGTT AATTCAGGGA ATAAATGTTT GCAAGGCAGG 60

AATGGTAAGG AACACCTCTT ACTACCATGA AGTTCAAAAA CAATCACAAA TAGGGCAGGC 120

TCCCTGACTG CCTCCACACC TCACAGTTTA TCATGCATTC GTGTATTTAG CACAGACTAG 180

ACAGATTTTT ACTTTATAAC ACTTTGTATT CATTCATTTT CCAACGTGCT TATTCCGGTT 240

CAGGGTCTTG GGTGGGGTGG CCAGAGCCAC CTGGCAGCTC AGGGCGCCAG GCGGGACCAG 300

CCCTGGACAG ACGCCACCAT CCTATTGCAG AGCCACTAAC ACCCACACTC ACTCAGACTG 360

GGCCCACGTA GATACATCCT CAGTTGGCTT AATGTGCAAG CTTTGGGATG TGGAAGGAA 419

(2) INFORMATION FOR SEQ ID NO:20:

(i) SEQUENCE CHARACTERISTICS:
(A) LENGTH: 527 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:
(A) ORGANISM: Homo sapiens
(D) DEVELOPMENTAL STAGE: fetal
(F) TISSUE TYPE: Liver

(vii) IMMEDIATE SOURCE:
(A) LIBRARY: lambda charon 4a
(B) CLONE: pF17BB2A

(viii) POSITION IN GENOME:
(A) CHROMOSOME/SEGMENT: 8
(B) MAP POSITION: 8p12

(ix) FEATURE:
(A) NAME/KEY: repeat_region
(B) LOCATION: 1..527

(xi) SEQUENCE DESCRIPTION: SEQ ID NO:20:

```
CTGGATCAGT GGTCCTCAGT CTTTTTTGCA CCAGGGACCA GTTTTGTAAA GATAGCTTTT     60

CCACGGACAG AGGGAGGGGA GATAGTTTCG GGATGATTCA AGAGGATTAC ATTTATTGTG    120

CACTTTATTT ATATTACTAT TACATTGTAT TATATAATGA AATAATGGTA TGACTCAGCA    180

TAATGTAGAA TCAGTGGGAA CCCTGAGCTT GTTTTCTTAT AACTAGATGG TCCCATCTGG    240

GGGTTATGGG AGACAGCGAC AGATCATCAG GCATTAGATT CTTATAAGGA GTGCACAACC    300

TCAATCCCTC GCATGTACAG TTCACAATGG GGTTTGCACT CCTATGAGAA TCTAGTGCCA    360

CTGCTGATCC AACCGGAGGT GCAGCTCAGG CAGTAATGCG AGTGATGGGG AGCGGCTGTA    420

AACACAGATG AAGCTTCACT GGCTCTCCAG CCACTCACCT CCTGCTGCGC AGCCTGGTTC    480

CTAACAGGCC ACGGACAGAT ACCAGCCCAT GGCCCCAGGG CCGGGGA                  527
```

(2) INFORMATION FOR SEQ ID NO:21:

(i) SEQUENCE CHARACTERISTICS:
(A) LENGTH: 142 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:
(A) ORGANISM: Homo sapiens
(D) DEVELOPMENTAL STAGE: fetal
(F) TISSUE TYPE: liver

(vii) IMMEDIATE SOURCE:
(A) LIBRARY: lambda charon 4A
(B) CLONE: FIXlambda61

(viii) POSITION IN GENOME:
(A) CHROMOSOME/SEGMENT: X
(B) MAP POSITION: long arm

(ix) FEATURE:
(A) NAME/KEY: repeat_region
(B) LOCATION: 1..142

(xi) SEQUENCE DESCRIPTION: SEQ ID NO:21:

```
TCAGAATCAC CTGGGAACGT AGAAATGCAA ATTCTCCTGC TCTACACTAG ACCTACCAAA    60
TCAGAATATC TAGGGGGTGG GGCCCAGCAG TCTGTGCGCA AACAAGCACT GCAGGTGATT   120
TTGATGCACA TTATAGTTTG AA                                            142
```

## REFERENCES


1) J. Collins & B. Hohn(1978). *Proc. Natl. Acad. Sci. U.S.A.* **75**: 4242-4246.
2) D.T. Burke, G.F. Carle & M.V. Olson(1987). *Science* **236**: 806-812.
3) W.R. Jelinek & S.R. Haynes(1982). *C.S.H.S.Q.B.* **46**: 1123-.
4) M.F. Singer(1982). *Int. Rev. Cytology* **76**: 67-112.
5) Carine,K., Solus J., Waltzer, E., Manch-Citron, J., Hamkalo, B.A., and Scheffler,I.E.(1986). *Som. Cell Mol. Genet.* **12**: 479-491.
6) Solus, J., Jaquemin-Sablon, A., Carine, K., Waltzer E., and Scheffler, I.E.(1988). Som. Cell Molec. Genet. **14**: 381-391.
7) Jurka, J. (1990) *Nuc. Acids Res.* **18**, 137-141.
8) Yanisch-Perron, C., Vieira, J., and Messing, J. (1985) *Gene* **33**: 103-119.
9) Kariya, Y., Kikuya. K., Hayashizaki, Y., Himeno, S., Tarui, S., and Matsubara, K. (1987) *Gene* **53**,1-10.
10) Sanger, F., Nicklen, S., and Coulson, A.R. (1977) *Proc. Natl. Acad. Sci.* USA**74**,5463-5467.
11) Brunner, A.M., Schimenti, J.C., and Duncan, C.H. (1986) *Biochemistry***25**,5028-5035.
12) Tabor, S., and Richardson, C.C. DNA (1987) *Proc. Natl. Acad. Sci. U.S.A.***84**,4767-4771.
13) Saiki, R.K., Gelfand, D.H., Stoffel, S., Scharf, S.J., Higuchi, R., Horn, G.T., Mullis, K.B., and Erlich, H.A. (1988) *Science***239**,487-491.
14) Thomas, P.S. (1980) *Proc. Natl. Acad. Sci. USA* **77**: 5201-5205.
15) Southern, E.M. (1975) *J. Mol. Biol.***98**, 503-517
16) Benton, W.D., and Davis, R.W. (1977) *Science* **196**,180-182.
17) Feinberg, A.P., and Vogelstein, B. (1983) *Anal. Biochem.* **132**, 6-13.
18) Wahl, G.M., Lewis, K.A., Ruiz, J.C., Rothenberg, B., Zhao, J., and Evans, G.A.(1987). *Proc. Nat. Acad. Sci.***84**: 2160-2164.
19) T.P. Yang, S.K. Hansen, K.K. Oishi, O.A. Ryder & B.A. Hamkalo(1982). *Proc. Natl. Acad. Sci. USA* **79**: 6593-6597.
20) Miller, D.A., Sharma, V., & Mitchell, A.R.(1988). *Chromosoma* **96**: 270-274.
21) H.C.Birnboim & J.Doly(1979). Nucl. Acids Res. **7**: 1513-.
22) P.W.J. Rigby, M. Dieckmann, C. Rhodes & P. Berg(1977) *J. Molec. Biol.* **11**: 237-251
23) Feinberg, A.P., and Vogelstein, B.(1984). Anal. Biochem. **137**: 266-267.
24) Kaplan, D.J. & Duncan, C.H. (1990) *Nuc. Acids Res.* **18,** 192.
25) Degen, S.J.F., Rajput, B., and Reich, E. (1986) *J. Biol. Chem.* **261**, 6972-6985.
26) Yoshitake, S., Schach, B.G., Foster, D.C., Davie, E.W.,.and Kurachi, K. (1985) *Biochemistry***24**,3736-3750.
27) Mullis, L.B. and Faloona, F.A. Specific Synthesis of DNA in vitro via a polymerase-catalyzed chain reaction. Methods in Enzymology **155**:335-350 (1987).


## Claims

1. A method of detecting overlaps among cloned DNA molecules, said method including the steps of: hybridizing a labeled low frequency repetitive sequence probe with cloned DNA molecules, at least some of the cloned DNA containing overlaps including the low frequency repetitive sequence; and identifying molecules or fragments of molecules containing the low frequency repetitive sequences as including DNA from the same genomic location.

2. A method as set forth in claim 1 wherein said identifying step is further defined as isolating as a subset the DNA molecules including the low frequency repetitive sequences; enzymatically digesting the DNA molecules to fragments of the DNA molecules; isolating fragments; hybridizing the fragments with the low frequency repetitive sequence probe; and correlating the common sized fragments including a low frequency repetitive sequences as a high probability of containing overlapping genomic locations.

3. A method as set forth in claim 2 wherein said digesting step is further defined as digesting the DNA molecules with various enzymes to different sets of fragments for measuring the probability that same size fragments from different enzymatic digestions containing the low frequency repetitive sequences are overlapping genomic locations.

4. A method as set forth in claim 1 wherein said identifying step is further defined as sequencing the genetic material of the molecules or fragments to specifically identify common genomic locations.

5. A method as set forth in claim 1 wherein said identifying step is further defined as use of the polymerase chain reaction to identify locations of low frequency repeats.

6. A method as set forth in claim 1 further including the step of screening several clones in parallel with several labeled low frequency repetitive sequence probes; identifying molecules or fragments containing two or more low frequency repetitive sequences; and ordering the fragments sharing common genomic locations identified by molecules or fragments sharing different low frequency repetitive sequences with different molecules or fragments.

7. A probe of nucleic acid for detecting overlaps among cloned DNA molecules comprising: a purified and labeled nucleic acid consisting of a sequence of base pairs of DNA repeated about 300 to 10,000 times in the human genome, said sequences having substantially random distribution throughout the human genome, said probe being capable of detecting specific sites in human genomic DNA.

8. A probe as set forth in claim 7 wherein said probe is a double strand of DNA.

9. A probe as set forth in claim 7 wherein said probe includes a radioactive label.

10. A probe as set forth in claim 7 wherein the DNA sequence of the probe is LF1:

```
        10         20         30         40         50         60
TGCATCTCTT AATGGGGATG CGTTCTGAAA ATGAGCCCTT AGGCAGTTTT ATTGTGTGAG
        70         80         90        100        110        120
CCTCATAGAG TGCACTTATG CAAACTAGAT GGTATAGCCT ACACACACCT AGGCTTTGTG
       130        140        150        160        170        180
ATAGAGCCTA CTGCTCGTAG GCTACAAGCT GTACAGCATG TTACTGTACT GAATACCATA
       190        200        210        220        230        240
GGCAGTAACA ATGGTAAGTA TTTGTGTATC TATACATAGA AATGGTTAAG TAAAAATATA
       250
GAATAGG
```

deposited at the ATCC, having designator 68254 (strain name XL1/PCDLF1).

11. A probe as set forth in claim 7 wherein the DNA sequence of the probe is LF2:

```
        10         20         30         40         50         60
TCCATGTACT CAACATATAC TCAAGTCCTG CAGTCAGCCC CACAGAACCT GCATATATGG
        70         80         90        100        110        120
CTAGTTTGCA TCCTGCAAAT ATTGTATTTT CAATCCACAT TTGATTGAAA AAGTCTGCAT
       130        140        150        160        170        180
GTAAGTCGAC CTGTGCAGTT CAAACCCATG TTGTTCAAGG GTCAACTATG TATGAAAACA
       190        200        210
TTTCTCTTCA GTAAGTACTT AGAAGTGGAA
```

deposited at the ATCC, having designator 68255 (strain name XL1/pCDLF2.

**12.** A probe as set forth in claim 7 wherein the DNA sequence of the probe is LF3:

```
        10         20         30         40         50         60
TCCTAGGCCT TCGCATTTAC TCACCACTCA CTGACTCAGT CAGAGCAACT TCCAGTCCTG
        70         80         90        100
CAAGCTCCAT TTATGGTAAG TACCCTATAC AGGTGTACCA TT
```

deposited at the ATCC, having designator 68256 (strain name XL1/PCDLF3).

**13.** A probe as set forth in claim 7 where in the DNA sequence ot the probe is LF4:

```
        10         20         30         40         50         60
GGATGATGGC GGCCATAATA GTGGTGGGTT AATTCAGGGA ATAAATGTTT GCAAGGCAGG
        70         80         90        100        110        120
AATGGTAAGG AACACCTCTT ACTACCATGA AGTTCAAAAA CAATCACAAA TAGGGCAGGC
       130        140        150        160        170        180
TCCCTGACTG CCTCCACACC TCACAGTTTA TCATGCATTC GTGTATTTAG CACAGACTAG
       190        200        210        220        230        240
ACAGATTTTT ACTTTATAAC ACTTTGTATT CATTCATTTT CCAACGTGCT TATTCCGGTT
       250        260        270        280        290        300
CAGGGTCTTG GGTGGGGTGG CCAGAGCCAC CTGGCAGCTC AGGGCGCCAG GCGGGACCAG
       310        320        330        340        350        360
CCCTGGACAG ACGCCACCAT CCTATTGCAG AGCCACTAAC ACCCACACTC ACTCAGACTG
       370        380        390        400        410        420
GGCCCACGTA GATACATCCT CAGTTGGCTT AATGTGCAAG CTTTGGGATG TGGAAGGAA
```

deposited at the ATCC, having designator 68257 (strain name XL1/PCDLF4).

**14.** A probe as set forth in claim 7 wherein the DNA sequence of the probe is LF5:

```
        10         20         30         40         50         60
TTCCAGGCCA TAGGTAGATA AGAGACAAAA GGCTGTATTC TGAGTCCTTG ATCAGCTTTT
        70         80         90        100        110        120
CACTGAACAC ACAATTGAGT CTGGCTCAGT TCATCTGCAT TTTTACATAA AAAATAGGGC
       130        140        150        160        170        180
AGAGGAAGCA ATCAGATACG CATTTGTCTC AGATGAGCAG AGGGATGATT TTCTGTCCTG
       190        200        210        220        230        240
CACCTGGGAA GATAAGCTAT CCATTTACAA TGCCAAGGTG AAAGTCAACA GAACTGTTTT
       250        260        270        280        290
AGGGTAAAGA TCTTTAGGCC TGCAAGGAAT GTCCTTGTAG AGAATT
```

deposited at the ATCC, having designator 68258 (strain name XL1/PCDLF5).

**15.** A probe as set forth in claim 7 wherein the DNA sequence of the probe is LF6:

```
        10         20         30         40         50         60
CTGGATCAGT GGTCCTCAGT CTTTTTTGCA CCAGGGACCA GTTTTGTAAA GATAGCTTTT
        70         80         90        100        110        120
CCACGGACAG AGGGAGGGGA GATAGTTTCG GGATGATTCA AGAGGATTAC ATTTATTGTG
       130        140        150        160        170        180
CACTTTATTT ATATTACTAT TACATTGTAT TATATAATGA AATAATGGTA TGACTCAGCA
       190        200        210        220        230        240
TAATGTAGAA TCAGTGGGAA CCCTGAGCTT GTTTTCTTAT AACTAGATGG TCCCATCTGG
       250        260        270        280        290        300
GGGTTATGGG AGACAGCGAC AGATCATCAG GCATTAGATT CTTATAAGGA GTGCACAACC
       310        320        330        340        350        360
TCAATCCCTC GCATGTACAG TTCACAATGG GGTTTGCACT CCTATGAGAA TCTAGTGCCA
       370        380        390        400        410        420
CTGCTGATCC AACCGGAGGT GCAGCTCAGG CAGTAATGCG AGTGATGGGG AGCGGCTGTA
       430        440        450        460        470        480
AACACAGATG AAGCTTCACT GGCTCTCCAG CCACTCACCT CCTGCTGCGC AGCCTGGTTC
       490        500        510        520
CTAACAGGCC ACGGACAGAT ACCAGCCCAT GGCCCCAGGG CCGGGGA
```

deposited at the ATCC, having designator 68259 (strain name XL1/PCDLF6).

**16.** A probe as set forth in claim 7 wherein the DNA sequence of the probe is LF7:

```
        10         20         30         40         50         60
TCAGCTCTCC TTGTGAGTTC CACAGCCACA GATTCAATTA ACTGCAGATC AAAAATATTC
        70         80         90        100        110        120
AAGAAAAAAA TGGATGGTTC GATCTCTACT GAACATGTAC AGACTCTTTT ATCTTTCATT
       130        140        150        160        170        180
ATTCCCTAAA CAATACAGCA TAACAACTAT TTACATAGCA TTTACATTGT ATTAGCTATT
       190        200        210        220        230        240
AAGAGAAACC TAGAGATGAT TTAAAGTACA AAGGAGGATG TGTTTAGGTT ATATGCAAAT
       250        260        270        280        290        300
AGTAAGCCAT TTTTATATCG GAGACTTGAG CATCCACAGA TCTTGATATT TGCAGGGGGT
       310        320        330
CTTGCCACCA ATTTTCCATG GATACTGAGG AA
```

deposited at the ATCC, having designator 68260 (strain name XL1/PCDLF7).

**17.** A probe as set forth in claim 7 wherein the DNA sequence of the probe is LF8:

```
        10         20         30         40         50         60
TCAGAATCAC CTGGGAACGT AGAAATGCAA ATTCTCCTGC TCTACACTAG ACCTACCAAA
        70         80         90        100        110        120
TCAGAATATC TAGGGGGTGG GGCCCAGCAG TCTGTGCGCA AACAAGCACT GCAGGTGATT
       130        140
TTGATGCACA TTATAGTTTG AA
```

deposited at the ATCC, having designator 68261 (strain name XL1/PCDLF8).

**18.** A probe as set forth in claim 7 wherein the DNA sequence of the probe is LF12:

```
        10         20         30         40         50         60
CAGTGGCACC CAGCCCCATT TTAACCATTT TTAAGTGGAC AGTTCACTGA CATTAAGTAC
        70         80         90        100        110        120
TACACATTCG CATTGTTGTG CCACCATCCC CACCATCCAT CTCCAGAACT TTTTTATCTT
       130        140        150        160        170        180
GTGAAACTAC GACTCTATAC TCCTTTAACA ATAACTCTCC ATTCTACCCT CTCCACAGCC
       190        200        210        220        230        240
CCTAGCAACT ACCATTCTAC TTTCTGTGTC TATAAATTTG ACTATTCTAA GTACCTCATA
       250
TGA
```

deposited at the ATCC, having designator 68436 (strain name XL1/pCDLF12).

**19.** A probe as set forth in claim 7 wherein the DNA sequence of the probe is LF15:

```
        10         20         30         40         50         60
CAGCTGTACT GGGCTGATAA GGGCCAATTC TAATGGGCTC ATCTTAACTT GATGACATCC
        70         80         90        100        110        120
ACAAAGTCTC TGTTTCCAAA TAAGGTCACA TTCACAGGCA CCAGAAGGTA GGGCTTCAAT
       130        140        150        160        170
ATGCATTTTG GGCGACAGAC TTCACCCACT GACAGCCATA TGTGTTCTTT ACAGTTG
```

deposited at the ATCC, having designator 68555 (strain name XL1/PCDLF15).

**20.** A probe as set forth in claim 7 wherein the DNA sequence of the probe is LF16:

```
        10         20         30         40         50         60
CAGCTGCATC TGGCCTAAAT CCTAATGTAA ACTATGAATT TTGGGTGATA ATGATGTGTA
        70         80         90        100        110        120
AATATAGGTT CATCAATTGT AACAAAGGTA CTACTTTGGT TCAGATGTTG ATAGTGGATA
       130        140        150        160
AGGCTCTGCA TGTATGTCTA GGAAAGGAGT ATATGGGTTA TCTCTG
```

deposited at the ATCC, having designator 68556 (strain name XL1/PCDLF16).

**21.** A probe as set forth in claim 7 wherein the DNA sequence of the probe is LF17:

```
        10         20         30         40         50         60
GGCCCACCCT ACTCCAGTAC GACCTCATCT GGGTTAATTC CTCGTGTGAT GGCCCTGTTT
        70         80         90        100        110        120
CCAAATGGTC ACAAACTGAG GTTCTAGGGG GTTAGGTCTT GAACGTATGA ATTTTGGGGA
       130        140        150        160
AATATGATGT ACCCCATAAC ACAAGGCATT TGAGACCTTA TTG
```

deposited at the ATCC, having designator 68557 (strain name XL1/PCDLF17).

**22.** A probe as set forth in claim 7 wherein the DNA sequence of the probe is LF18:

```
        10         20         30         40         50         60
CAGCTGTGCC CAGCCTGTTT TTAGTCTTAT AGTGGCATTC TGGGGCTCTT GTGTTGTAGT
        70         80         90        100        110        120
GATATTGAGT TGCACATTGT CTTAGTTTCC TAGGCTACCT TAACGAAGTA CCACAGACTG
       130        140        150        160        170        180
AGTTACTTAA ACAACAGAAA CTTATTTTTG TACAATTCTG GAGACTGGAA GTCAAGATCA
       190        200        210        220        230        240
AGGTGCTGGA GGGATTAGTT TCTCTCGAGG TCTCCTTGGC TTGTAGATGC TGTCTTTTTC
       250        260        270        280
CTGTGTCTAC ACAATGCCTT CCTCTGTGCA TGTCTGTGA
```

deposited at the ATCC, having designator 68558 (strain name XL1/PCDLF18).

**23.** A probe as set forth in claim 67 wherein the DNA sequence of the probe is LF19:

```
        10         20         30         40         50         60
TGATTAGATG GTGTCCACTC AGATTAAGGG TGGGTCTGCC TTTCCCCAGC CCACTGAGTC
        70         80         90        100        110        120
CAATGTTAAC CTCCTTTGGC AACACCCTCA CAGACACACC CAGGATCAAT ACTTTGTAAC
       130        140        150        160        170        180
CTTCAATCTA ATAAAGTTGA CAGTATTAAC CATCACACCT GCCTAAGTAA AGATTAAATG
       190        200
AGATGATCAT CACAACGCAT ATTTG
```

deposited at the ATCC, having designator 68559 (strain name XL1/PDCLF19).

**24.** A probe as set forth in claim 7 wherein the DNA sequence of the probe is LF20:

```
        10         20         30         40         50         60
CTTGTAAGCA GGGCTTTCAA AAGGCAGCAT TTGCATCTGC TATGTTAACT TTTTAATACG
        70         80         90        100        110        120
CTTTGGGCAG AGAACTGTAA GAGATCCCAG AGGACATTTG ACAATTTCTG GAGACATTTC
       130        140        150        160        170        180
TGTTGTCGTA ACTGGGAGGT CCACAGACAT ACATTGGATA GAAGCCAGGA ATGCTGCTAA
       190        200        210        220        230        240
ATGCTATGCA GTCCACAGAA CAGCCCCCAT CCCCTCAACC AAAAATTATG TGGCCCAAAA
       250        260        270        280        290        300
TGTCAGTAGT GTCAAGGTGG AGAAACTCTC GTCTAGACCT ACCCTAACAG AATTTAACAA
       310        320
CAAGCCTGGA AGGATCAAGT TGA
```

deposited at the ATCC, having designator 68560 (strain name XL1/PCDLF20).

**25.** A probe as set forth in claim 7 wherein the DNA sequence of the probe is LF21:

```
        10         20         30         40         50         60
AATTCTGACA CTGTCTACCT GGAGATAGCA TCAAATCCCA GGGTTGAGGT CTCAGTCTGA
        70         80         90        100        110        120
CAAGACTGCA CCCCTCTTCA GACACAGTAG CATGTCTGGG CCTCTGGAAC TTCTGACCAA
       130        140        150        160        170        180
CTGGCTTCAC ATTGGGGTAA CCAGACTTCG TATTTATGTT CAATTAATTT GCTAGAGTGG
       190        200        210        220        230        240
CTCACAAAAC TCAGAGAAAT ACATTTACTG GTTAATTATA AAGGATATTT ATGCCTTTAT
       250        260        270        280        290        300
ATTCCTTGCA CTTTCAATTC ATCTGTTTCT TTCAGGCACAT CTTTATAGA A
```

deposited at the ATCC, having designator 68561 (strain name XL1/PCDLF21).

**26.** A probe as set forth in claim 6 wherein the DNA sequence of the probe is LF22:

```
        10         20         30         40         50         60
CAGCCGCGCC CGGCCTATAT ATATATTTCT GGATGACGTG AGATATTTTG ATACAGGCAT
        70         80         90        100        110        120
GCAATCGATA ATAATCACAT CAAGGTAAAT GAGGTCTCCA TCCCCGCAAT CATTTATCCT
       130        140        150        160        170        180
TTCTGTTACA AATGATCCAG TCTACTCTTG TAG
```

deposited at the ATCC, having designator 68562 (strain name XL1/PCDLF22).

Table 1

Repetitions of Individual LF sequences in the human genome.

| Prcbe | Repetiticn per haplcid gencme | Number cf times in GenBank | Number cf times expected in GenBank |
|---|---|---|---|
| LF1 | 650 | 3 | 1.0 |
| LF2 | 560 | 2 | 0.8 |
| LF3 | 2850 | 1 | 4.3 |
| LF4 | 850 | 2 | 1.3 |
| LF5 | 1400 | 3 | 2.1 |
| LF6 | 6700 | 5 | 10.0 |
| LF7 | 2500 | 4 | 3.7 |
| LF8 | 1400 | 2 | 2.1 |
| LF12 | 3850 | 4 | 5.8 |
| LF15 | 1100 | 5 | 1.6 |
| LF16 | 420 | 2 | 0.6 |
| LF17 | 1100 | 5 | 1.6 |
| LF18 | 7000 | 5 | 10.5 |
| LF19 | 3350 | 1 | 5.0 |
| LF20 | 300 | 1 | 0.4 |
| LF21 | 850 | 1 | 1.3 |
| LF22 | 2500 | 2 | 3.7 |

In this table the repetition frequency is calculated as described in the text. The figure "Number of times in GenBank" refers to the number of times this sequence was found in a search of GenBank(Release #65) DNA Databank. The number of times the sequence is expected in the Genbank database is the repetitions/haploid genome times the fraction of the genome represented in the database(approximately 0.0015).

Table 2

| LF sequences present at the centromere region of human chromosome 1. | | | | | |
|---|---|---|---|---|---|
| sequence probe | #sites (estimated in genome) | #clones (detected in mini) | #sites (expected in mini) | #sites (found in mini) | #overlaps (detected in mini) |
| LF1 | 650 | 10 | 0.8 | 5 | 3 |
| LF2 | 560 | 3 | 0.7 | 2 | 1 |
| LF3 | 2850 | 1 | 3.5 | 1 | 0 |
| LF4 | 850 | 3 | 1.0 | 3 | 0 |
| LF5 | 1400 | 9 | 1.7 | 7 | 2 |
| LF6 | 6700 | 15 | 8.3 | 12 | 2 |
| LF7 | 2500 | 13 | 3.1 | 10 | 1 |
| LF8 | 1400 | 13 | 1.7 | 7 | 2 |
| LF12 | 3850 | 22 | 4.8 | 18 | 1 |
| LF15 | 1100 | 5 | 1.3 | 5 | 0 |
| LF16 | 420 | 4 | 0.5 | 2 | 0 |
| LF17 | 1100 | 13 | 1.3 | 10 | 2 |
| LF18 | 7000 | 71 | 8.7 | 39 | 7 |
| LF19 | 3350 | 10 | 4.1 | ≦10 | n d |
| LF20 | 300 | 8 | 0.3 | ≦8 | n d |
| LF21 | 850 | 3 | 1.0 | ≦2 | n d |
| LF22 | 2500 | 10 | 3.1 | ≦8 | n d |
| Table 2 summarizes the data obtained from the hybridization of the LF sequence probes described in this manuscript to cloned samples of human DNA from the centromere region of chromosome 1.<br>* calculated from #sites estimated in genome x 0.00125(fraction of human DNA in the hybrid X12.1.3. | | | | | |

Fig-1

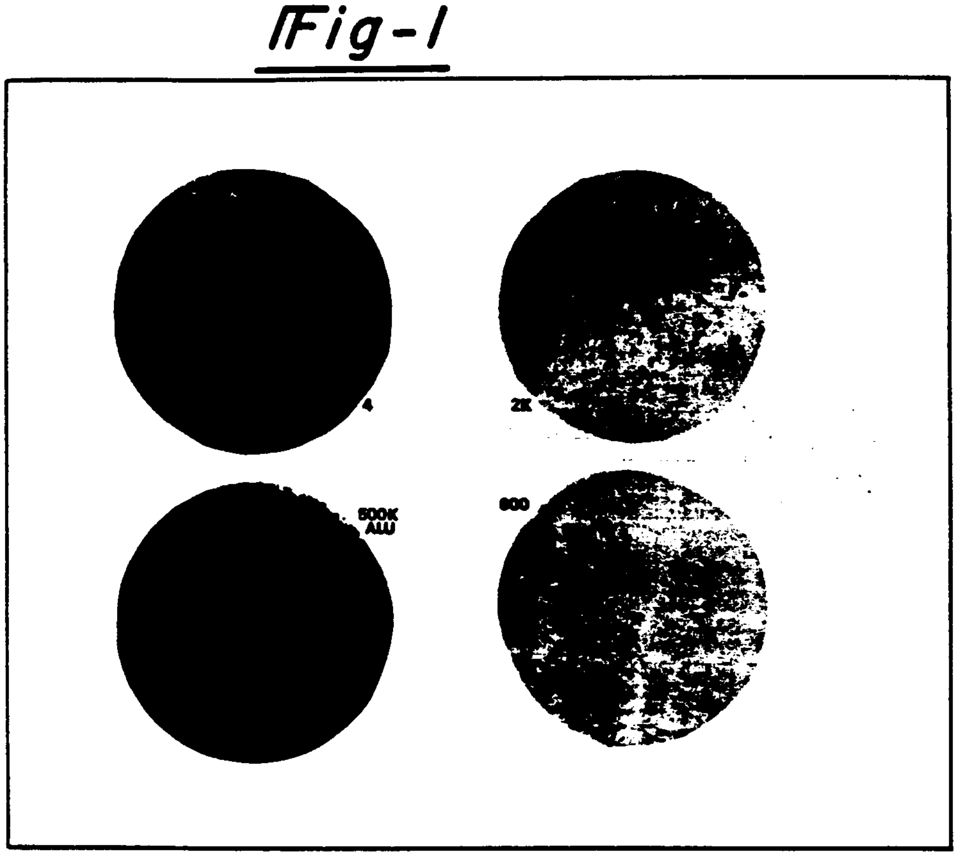


Fig-2

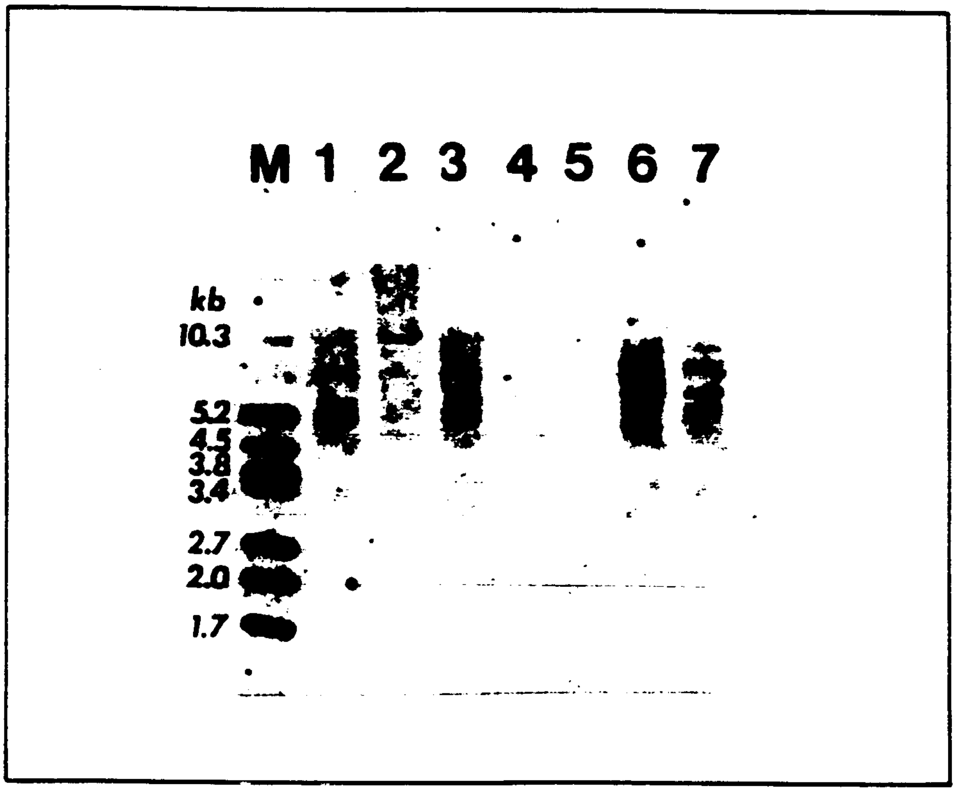

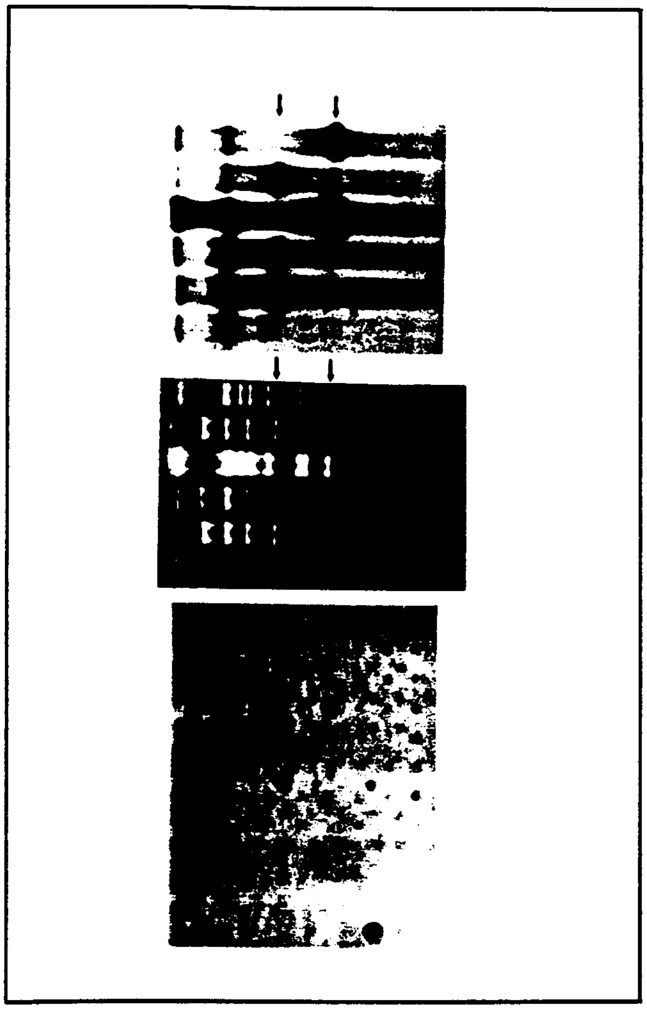

*Fig-3*

*Fig-4*

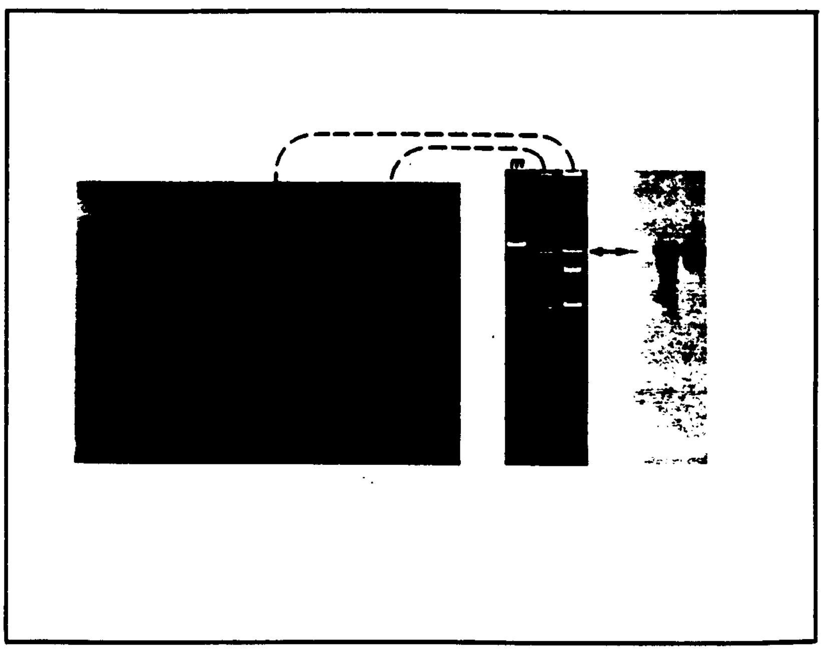

Figure 5